# EUROPEAN PATENT SPECIFICATION

(11) **EP 4 504 146 B1**
(45) Date of publication and mention of the grant of the patent: **17.12.2025**
(21) Application number: 23718622.6
(22) Date of filing: 03.04.2023
(51) Int. Cl.: A61K 9/00, A61K 9/14, A24F 23/02, A61K 31/465, A61K 47/02, A61P 25/14, A61P 25/16, A61P 25/28, A61P 25/34, A24B 13/00

(54) **ALKALINE INTRAORAL FORMULATIONS**
ALKALISCHE INTRAORALE FORMULIERUNGEN
FORMULATIONS INTRABUCCALES ALCALINES

(30) Priority: 01.04.2022 GB 202204804; 15.06.2022 GB 202208807
(43) Date of publication of application: 12.02.2025
(73) Proprietor: EMPLICURE AB (publ), 752 28 Uppsala (SE)
(72) Inventor: ENGQVIST, Håkan, 75229 Uppsala (SE); LÖÖF, Jesper, 74381 Bälinge (SE)
(74) Representative: Potter Clarkson
(86) International application number: PCT/EP2023/058713
(87) International publication number: WO 2023/187224

(56) References cited:
- WO-A1-2015/193379
- WO-A1-2019/048880
- WO-A1-2019/110075
- WO-A1-2020/157280
- WO-A1-2022/219310
- CN-A- 103 284 318
- CN-A- 107 259 635
- US-A1- 2011 014 132
- US-A1- 2018 084 820
- US-A1- 2021 169 123
- SOPAPHAN KANJANABAT ET AL: "Preparation and Characterization of Nicotine-Magnesium Aluminum Silicate Complex-Loaded Sodium Alginate Matrix Tablets for Buccal Delivery", AAPS PHARMSCITECH, SPRINGER NEW YORK LLC, US, vol. 12, no. 2, 19 May 2011 (2011-05-19), pages 683 - 692, XP019925563, ISSN: 1530-9932, DOI: 10.1208/S12249-011-9633-Y

## Description

### Field of the Invention

The invention relates to new formulations for intraoral delivery of nicotine. The invention also relates to a method of manufacturing nicotine formulations suitable for intraoral delivery. The formulations may be employed in aiding smoking cessation, treating nicotine dependence or alleviating the symptoms of nicotine dependence. The formulations may also be used in recreational use products.

### Background

Various nicotine replacement therapy products are commercially available, including patches, gums, lozenges, nicotine pouches, sublingual tablets, inhalers and nasal sprays (U. Wadgave et al., Int J Health Sci (Qassim), 2016 Jul; 10(3): 425-435). Nicotine replacement therapies such as these are useful in helping tobacco users to overcome difficulties arising from nicotine withdrawal as they attempt to reduce their exposure to tobacco products, particularly cigarettes.

Some nicotine delivery systems are also used by individuals to provide pleasurable sensations resulting from nicotine uptake. Intraoral products that are used in this way include nicotine pouches and snus, the latter being particularly popular in Norway and Sweden. Snus is a tobacco-containing product that is typically provided as either loose snus or portion snus. Snus products are also a convenient and safer alternative to cigarettes as they offer a source of nicotine which does not expose the user to some of risks associated with cigarette smoking, such as harmful substances found in cigarette smoke (e.g. carbon monoxide, benzene and formaldehyde) and in the cigarette itself (e.g. vinyl chloride that is used in cigarette filters).

Loose snus is a moist powder which can be portioned and packed into a cylindrical or spherical shape with the fingertips or a purpose-made device. The shaped mass of snus is then placed under their upper lip. Over time, the demand for loose snus has been replaced by portioned varieties, which benefits users due to easy handling and their more discreet nature.

Portion snus is packaged as a moist powder in small teabag-like sachets, which are also intended for positioning under the upper lip. Like snus, portion snus is typically held in the mouth for a period lasting between five minutes and one hour and nicotine is released throughout this time.

Portion snus is available in three different sizes: mini, normal/large (most common) and maxi. Mini portions typically weigh close to 0.5 g, normal (large) portions weigh around 0.8 to 1 gram, and maxi portions weigh up to 1.7 g. Some manufacturers also offer the choice of "regular" and "long" versions of the normal size sachet, which are similar in content weight. These long portions differ from traditional sachets in that they are slimmer but longer, in order to fit against the gums more comfortably. Tobacco-containing snus products often require refrigerated storage conditions, primarily to slow the drying of the tobacco. Refrigeration is also commonly used for tobacco-free nicotine pouches products in order to improve the stability of the product.

Nicotine pouches are white pre-portioned pouches containing either tobacco-derived nicotine or synthetic nicotine, potentially in salt form, but no tobacco leaf, ground tobacco (i.e. dust), or tobacco stem. They are described as either similar to snus, or a tobacco-free version of snus. Non-tobacco-based nicotine pouches are commercially available, and one of the first examples of this was ZYN^{®}, a product marked by Swedish Match^{®} (N. Plurphanswat et al., The American Journal on Addictions, 29: 279-286, 2020; Lunell E., et al., Nicotine & Tobacco Research, 2020, 1757-1763). A number of cellulose-containing commercial products are available that deliver nicotine. However, when placed in the mouth, the cellulose can interact with water to form a slimy lump in the consumer's mouth, which negatively affects the user's flavour experience and will therefore likely affect the release of nicotine.

Nicotine pouches have been approved by e.g. the Norwegian Medicines Agency for smoking cessation and are sold as a nicotine replacement therapy. Nicotine pouches are also increasingly used recreationally as an alternative to tobacco products such as cigarettes and snus. Nicotine pouches may be dry or moist. Pouches that are moist are generally feel more comfortable in the mouth, and often provide for quicker release of nicotine. Moist pouches typically contain glycerol.

To use a nicotine pouch, the user puts a pouch in contact with the inside surface of the mouth, typically between the upper lip and gum, behind the lower lip or against the cheek, and leaves it there while the nicotine and taste is being released, much like portion snus. The pouch is typically kept in the mouth for a period of from five minutes to one hour and are then disposed of when finished.

Fast-releasing oral nicotine products are disclosed in the international patent application with the publication no. WO 2019/110073 and WO 2019 / 110075. Pouches containing nicotine in free salt form are disclosed in US patent no. 9,161,908.

The delivery of nicotine through the oral mucosa (particularly sublingual and buccal mucosa) can be greatly enhanced by increasing the pH of the oral environment above the normal level, typically to around pH 8-9 (See Ciolino LA, McCauley HA, Fraser DB, Wolnik KA. J. Anal. Toxicol. 2001;25:15-25, and Tomar S. L. et al. Tobacco Control 1997;6:219-225). A pH regulating agent or suitable buffering agent may be incorporated into an intraoral product in order to achieve this pH increase. Commercially available nicotine pouches typically have a release profile in which a significant fraction of the nicotine is released in a short time and may lead to nicotine being swallowed and/or cause saturation of the mucosa. It has been speculated that this leads to a significant amount of the available nicotine being washed away by saliva and swallowed if the mucosa and immediately surrounding saliva is saturated. Controlling the total amount and rate of release of nicotine from an intraoral product to provide long lasting therapeutic and/or non-therapeutic effects presents further challenges. There is an unmet need for oral transmucosal (intraoral) products offering controlled release of nicotine and which also provide and maintain a suitably alkaline environment to ensure adequate uptake of nicotine into the bloodstream.

We have now devised a novel formulation of nicotine (or a salt thereof) suitable for intraoral delivery, and a method for its manufacture, that have advantageous properties and which solve one or more of the problems associated with nicotine formulations suitable for intraoral delivery.

The listing or discussion of an apparently prior-published document in this specification should not necessarily be taken as an acknowledgement that the document is part of the state of the art or is common general knowledge.

### Disclosure of the Invention

According to a first aspect of the invention, there is provided an intraoral formulation comprising a solid, porous chemically bonded ceramic system, nicotine or a salt thereof, and a pH regulating agent, wherein the chemically bonded ceramic system contains calcium and is formed in the absence of said pH regulating agent, and wherein the chemically bonded ceramic system is formed in the presence of nicotine or a salt thereof such that said nicotine or salt thereof is located in pores in the chemically bonded ceramic system.

These intraoral formulations are hereinafter referred to as "formulations of the invention".

We have advantageously found that for formulations of the invention that are prepared in this way, i.e. where a pH regulating agent is mixed with, or provided alongside, a combination of a solid, porous chemically bonded ceramic system and nicotine (or a salt thereof) but the chemically bonded ceramic system was formed in the absence of said pH regulating agent, it is possible to achieve a high degree of loading of nicotine (or a salt thereof) into the pores of the chemically bonded ceramic system whilst still enabling the product to deliver the nicotine through the oral mucosa at a satisfactory rate and to a satisfactory extent once in the mouth.

By the use of the term "chemically bonded ceramic system" (or similar) we refer to materials that are capable of being formed at room temperature or at slightly elevated temperature (e.g. less than about 200 °C). These systems act as carriers in the formulations of the invention as they contain pores within which the nicotine (or salt thereof) may be located.

The term "chemically bonded ceramic" typically refers to a system formed from a self-setting precursor material. Chemically bonded ceramic systems include non-hydrated, partly hydrated or fully hydrated ceramics. Therefore, in an embodiment of the disclosure, the porous solid is based on a ceramic material that is formed from a self-setting precursor ceramic. Non-limiting examples of chemically bonded ceramic systems include those formed from calcium phosphates, calcium sulphates, calcium silicates, calcium aluminates, magnesium carbonates and combinations thereof. Preferred chemical compositions include those based on chemically bonded ceramic systems, which following hydration of one or more appropriate precursor substances consume a controlled amount of water to form a network. Precursor substances that may be mentioned in this respect include CaOAl₂O₃, (CaO)₁₂(Al₂O₃)₇, (CaO)₃(Al₂O₃), (CaO)(Al₂O₃)₂, (CaO)₃(SiO₂), (CaO)₂(SiO₂), and, particularly, alpha-tricalcium phosphate, tetracalcium phosphate (Ca₄(PO₄)₂O), and calcium sulphate (e.g. calcium sulphate hemihydrate). These substances are all capable of reacting with water at room temperature to form a chemically bonded ceramic system. Water may be provided in liquid form, or the precursor substance may be exposed to a humid atmosphere for a sufficient amount of time for the material to cure and harden.

Other particular systems available are those based on aluminates and silicates, both of which consume a great amount of water. Phases such CA2, CA, C3A and C12A7, and C2S and C3S in crystalline or amorphous state (C= CaO, A =Al₂O₃, SiO₂ = S, according to common cement terminology) may be used, which are readily available. The calcium aluminate and/or calcium silicate phases may be used as separate phases or as mixtures of phases. The above-mentioned phases, all in non-hydrated form, act as the binder phase (the cement) in the carrier when hydrated. The liquid(water)-to-cement weight ratio is typically in the region of 0.2 to 0.5, preferably in the region of 0.3 to 0.4.

The water-to-cement ratio during manufacturing, particularly for chemically bonded ceramic systems formed from calcium aluminates and calcium silicates, is important for the pore size and pore volume. As an example, for a phase pure 1: 1 CA precursor the theoretic water-to-cement ("W/C") ratio that gives complete hydration and complete use of all water is about 0.4. If the W/C ratio is increased, any excess water present will result in an increased pore volume and, to some extent, increased pore size. For calcium aluminates and calcium silicates, the permissible range of water content is quite wide, i.e. it is possible to increase the W/C ratio far above the theoretic value needed for complete hydration and still have a hardened body with sufficient structural integrity.

Particular ceramic materials that may be employed include those based upon a sulphate, such as a calcium sulphate or a phosphate such as a calcium phosphate. Particular examples of such substances include alfa or beta phase calcium sulphate hemihydrate (end product calcium sulphate dihydrate), alkaline or neutral calcium phosphate (apatite) and acidic calcium phosphate (brushite).

The use of chemically bonded ceramic systems that contain calcium may be particularly beneficial for a user's oral health. Chemically bonded ceramic systems that are capable of releasing calcium (e.g. in the form of solubilised calcium ions) into saliva are capable of remineralisation and so can, for example, contribute to mineral growth on the surfaces of teeth. Materials that allow excess ions (e.g. calcium ions, hydroxide ions and possibly also phosphate ions) to diffuse into surrounding saliva under physiological conditions (e.g. at the pH and temperature typically found in the mouth) may be particularly suited for this, and such materials include chemically bonded ceramic systems formed from alpha-tricalcium phosphate and tetracalcium phosphate. Chemically bonded ceramic system that are formed from materials other than calcium phosphates but which are still capable of releasing calcium into saliva are also useful in this context as saliva can act as a source of phosphate ions, thus contributing to the growth of minerals, such as apatite, on teeth. Such materials include chemically bonded ceramic systems formed from calcium silicates, calcium aluminates and, particularly, calcium sulphate.

The intraoral formulations of the invention may contain any amount of carrier that is sufficient to hold and deliver the intended amount of nicotine in use. In one embodiment of the invention, the chemically bonded ceramic system is present at from about 40% to about 98% by weight of the intraoral formulation. The intraoral products described herein (e.g. nicotine pouches and sublingual and buccal tablets) typically have a mass not exceeding 2 grams. In an embodiment of the invention, the chemically bonded ceramic system is present at from about 40% to about 98% by weight of the intraoral product (e.g. nicotine pouches and sublingual and buccal tablets).

In the formulations of the invention, the chemically bonded ceramic system is formed in the presence of nicotine or a salt thereof such that said nicotine or salt thereof is located in pores in the chemically bonded ceramic system. Nevertheless, we also disclose formulations in which the chemically bonded ceramic systems used in the formulation of the invention is loaded with nicotine or a salt thereof by soaking the chemically bonded ceramic material in a liquid containing the nicotine or its salt, or through any other method which facilitates the drawing up of that substance into the pores of the chemically bonded ceramic material via capillary forces (including spraying, brushing, rolling, dip coating, powder coating, misting or vacuum enhanced loading). In one example, the pH regulating agent is not present during the process of loading the chemically bonded ceramic system with nicotine (or salt thereof). The pH regulating agent may then be added to the loaded chemically bonded ceramic system to provide the formulation of the invention. Alternatively, the pH regulating agent is added to the chemically bonded ceramic system prior to the nicotine.

Oral products containing the formulations of the invention, particularly nicotine pouches, may also be suitable for storage under ambient temperature without significant degradation of the components, e.g. the pH regulating agents, contained within. Avoiding the need for refrigerated storage is of clear benefit for manufacturers, retailers and users of these products.

The nicotine (or salt thereof) is present when the chemically bonded ceramic system is formed. This allows for greater control over the extent to which the pores in the chemically bonded ceramic system are filled with the nicotine, and so allows greater control over the release characteristics of the final formulation. In particular, this loading method enables the total amount of nicotine that is stored in, and released from, the chemically bonded ceramic system to be more reliably controlled.

As is hereinbefore described, the chemically bonded ceramic system is formed from a suitable precursor material, such as a calcium sulphate or a calcium phosphate, which is typically provided in a powdered form for hydration. The mean grain size of any precursor powder particles may be below about 500 µm, e.g. below about 100 µm, preferably between about 1 µm and about 30 µm. This is to enhance hydration. Such precursor material may be transformed into a nano-size microstructure during hydration (e.g. when brought into contact with liquid water or a humid atmosphere). This reaction involves dissolution of the precursor material and repeated subsequent precipitation of nano-size hydrates in the water (solution) and upon remaining non-hydrated precursor material. This reaction favourably continues until precursor materials have been transformed and/or until a pre-selected porosity determined by partial hydration using the time and temperature, as well as the H₂O in liquid and/or humidity, is measured.

Pore sizes in chemically bonded ceramic systems may be controlled by various techniques during the process of fabricating the carrier material network structure. A particular method that is suitable for use with the chemically bonded ceramic systems used in the present invention is the porogen leaching method which involves the use of a sacrificial phase during the formation of the chemically bonded ceramic system. A porogenic material may be included as part of the reaction mixture during the formation of the chemically bonded ceramic system in order to assist in the formation of pores within the final carrier material network. Porogenic materials include, for example, oils, liquids (e.g. water), sugars, mannitol etc. The porogenic material may then be removed from the carrier, e.g. by burning it away through heating during the curing process, or by dissolving it away using an appropriate solvent. Dissolving is usually achieved with water in order to avoid leaving residual amounts of a substance which may have deleterious effects on the formulation or adverse effects on the user. In such cases, the nicotine or salt thereof may subsequently be loaded into the pores in the chemically bonded ceramic system by any of the methods disclosed elsewhere herein. A porogenic material with a secondary function may also be used. For example, the porogenic material may act as a source of flavour, e.g. as a sweetener.

In such cases, it is not essential for the porogenic material to be removed from the carrier prior to use. Instead, some or all of the porogenic material may remain in the carrier, alongside the nicotine (or salt thereof), so that both substances can be released in the user's mouth. Porogenic materials that rapidly dissolve in water are particularly suited for use in this way. Still further, porogenic materials that slowly dissolve in water may be useful for controlling the release of the alkaline active pharmaceutical agent, for example when sustained-release is desired. Porogenic materials that may be used as sweeteners include sweetening agents known in the art, particularly mono-, oligo- and poly-saccharides; sugar alcohols such as mannitol, sorbitol, maltitol and xylitol; natural and synthetic sweeteners such as sucrose, glucose, dextrose, maltose, fructose, saccharin, aspartame, acesulfame K, sucralose, saccharin and cyclamates; and mixtures thereof.

Foaming methods may also be used to increase the pore sizes in chemically bonded ceramic systems. Such methods would be known to the skilled person and are particularly useful for forming carriers with larger pore sizes. One example of such a method involves preparing the carrier using carbonated water or water containing air bubbles. This may also be achieved without the use of foaming agents (e.g. Tween 80)

The total porosity of the chemically bonded ceramic system may be from about 10% to about 70%, such as from about 20% to about 40%. Porosities and average pore sizes may be measured by methods known to the skilled person, for example the mercury intrusion method, the BET (Brunauer, Emmet, and Teller) method, and N₂-adsorption techniques.

In a preferred embodiment of the invention, the nicotine (or salt thereof) is co-formedly interspersed in pores within the carrier material network. This means that, whatever process is employed to form the carrier, it must also necessarily form pores within which the nicotine is interspersed. Where the process by which the carrier is formed involves the use of porogens, the nicotine will also be interspersed within the pores arising directly from the use of said porogens. Chemically bonded ceramic systems are particularly suited for use in such embodiments as the process by which the carrier and its pore network is formed does not require very high temperatures, in contrast to sintered ceramics.

The nicotine (or salt thereof) may be mixed with precursor(s) to the chemically bonded ceramic system using a variety of techniques, such as dry powder mixing.

Alternatively, the nicotine (or salt thereof) and precursor(s) may be mixed by way of a sol-gel process, as a solution, or as a slurry, a paste or a putty of, for example, particles, granules or pellets of said precursor(s), in the presence of an appropriate liquid (e.g. an aqueous or organic solvent). This mixing step is followed by some sort of "curing" process to form the chemically bonded ceramic system, which comprises pores within which the nicotine resides. Chemically bonded ceramic systems that are formed in this way may be said to be pre-loaded with the nicotine.

Such pores are themselves a three-dimensional network of channels or voids within the solid network, containing (e.g. particles of) nicotine or a salt thereof.

Such pores may thus be essentially "secondary pores" formed by chemical interactions (e.g. "bonding") between the surfaces of primary particles of carrier (which may be porous in their own right (i.e. comprise "primary" pores). Such pores may, for example, result from exposure of such materials to one or more chemical reagents that cause a physical and/or chemical transformation (such as a partial dissolution) at, and subsequent physical and/or chemical bonding together of, those surfaces (which may in itself result as a consequence of some other physico-chemical process such as drying, curing, etc.), giving rise to said pores/voids.

In such instances, the chemical reagents may be mixed together with the nicotine prior to or during preparation of the chemically bonded ceramic system. However, such secondary pores are not necessarily formed in this way, and bonding together of primary particles of chemically bonded ceramic material may also be physical and/or mechanical, or may be formed during the production of a three-dimensional, chemically bonded ceramic network as described hereinbefore, in the presence of the nicotine.

Preferably, the precursors for the chemically bonded ceramic system are mixed with the nicotine (or salt thereof) prior to the hardening process taking place. The nicotine is then present at the moment when pore formation occurs with the result that the nicotine becomes located within the pores of the chemically bonded ceramic system. Excellent control over the total amount of nicotine loaded into the formulation can be achieved by processing in this way. Therefore, at least a portion of the nicotine (or salt thereof) is located within the pores of the (hardened) chemically bonded ceramic system. By this, we mean that at least 20% (e.g. at least 30%, at least 40%, at least 50%, at least 60%, at least 70%, or at least 80%) by weight of the nicotine (or salt thereof) is located within the pores of the chemically bonded ceramic system. In one embodiment, essentially all of the nicotine (or salt thereof) is located within the pores of the chemically bonded ceramic system. By this, we mean that at least 90% (e.g. at least 95%, preferably at least 98%) by weight of the nicotine (or salt thereof) is located within the pores of the chemically bonded ceramic system.

The amount of nicotine (or salt thereof) present in the formulations of the invention may vary considerably according to the intended form of the intraoral product and the intended release profile. For example, the formulation may comprise said nicotine or salt thereof in an amount of from 0.1% to 50% by weight of the formulation.

The formulations of the invention are intended for use in the intraoral delivery of nicotine, both for therapeutic and recreational purposes. The formulations offer an alternative to tobacco products (such as cigarettes and tobacco-containing snus) and can so be used to reduce a user's exposure to many of the potentially toxic components found in tobacco products. Nicotine is typically obtained from tobacco products, e.g. tobacco oil and other extracts. For example, the nicotine can be provided as a free base (e.g., as a mixture of nicotine free base and a porous particulate carrier such as microcrystalline cellulose), a nicotine salt (e.g., as nicotine tartrate or nicotine bitartrate or another organic acid salt of nicotine), as a resin complex of nicotine (e.g., nicotine polacrilex), or as a solvate or other suitable form.

Preferably, some or all of the nicotine is present in the formulations of the invention as a water-soluble form of nicotine. In this context, the term "water-soluble form" is understood as referring to a form of nicotine having a solubility in water of at least 10 g of salt per 100 mL water under ambient conditions, including temperature of 25°C, atmospheric pressure, and pH of 7. The nicotine present in the formulations of the invention is believed to exist as an (at least largely) amorphous material. The process by which the chemically bonded ceramic systems used in the present invention are formed involves hydration of suitable precursor materials in the presence of nicotine (or a salt thereof) followed by a hardening step. At the start of the manufacturing process, the nicotine or salt thereof is preferably provided in the form of a crystalline solid because crystalline solids of high purity can be readily sourced. During the process, the nicotine (or salt) is dissolved and/or diluted in the hydration liquid, and the subsequent drying step results in the precipitation of an amorphous nicotine product within pores in the chemically bonded ceramic system. This amorphous nicotine product may be a salt of nicotine. Powder X-ray crystallography can be used to detect the presence of crystalline components (such as nicotine salts), and other methods for detection and quantification of crystallinity and/or amorphicity are known in the art.

In one embodiment, the formulation of the invention is prepared using a salt of nicotine, such as a nicotine bitartrate salt (e.g. nicotine bitartrate dihydrate). Other salt forms that may be mentioned include nicotine ascorbate, nicotine aspartate, nicotine benzoate, nicotine monotartrate, nicotine chloride (e.g., nicotine hydrochloride and nicotine dihydrochloride), nicotine citrate, nicotine fumarate, nicotine gentisate, nicotine lactate, nicotine mucate, nicotine laurate, nicotine levulinate, nicotine malate, nicotine perchlorate, nicotine pyruvate, nicotine salicylate, nicotine sorbate, nicotine succinate, nicotine zinc chloride, nicotine sulphate, nicotine tosylate and hydrates thereof (e.g., nicotine zinc chloride monohydrate). For the avoidance of doubt, where a formulation of the invention is prepared using a salt of nicotine, the nicotine may be present in the final formulation as nicotine free base, a salt of nicotine (e.g. nicotine bitartrate) or a mixture thereof. Furthermore, the nicotine in the formulation may be largely (e.g. at least 70%) amorphous.

Snus pouches containing the formulations of the invention may also contain ground tobacco. However, in particular embodiments, the formulations of the invention do not contain ground tobacco. The nicotine (or salt thereof) in the formulations of the invention is believed to have an enhanced stability profile compared to commercially available formulations, i.e. compared to formulations in which nicotine is not stored within pores of a chemically bonded ceramic carrier of the sort disclosed here. Without wishing to be bound by theory, it is believed that the formulations of the invention prevent or slow the oxidation of nicotine, thus extending the shelf-life of the product.

The rate at which nicotine is absorbed into the body via the oral mucosa varies depending on the pH of the saliva. Tomar S. L. et al. (Tobacco Control 1997;6:219-225) reported that increased alkalinity promotes the absorption of nicotine and increases its physiological effects. A pH regulating agent is therefore provided in the formulations of the invention to enhance the rate and extent of transmucosal uptake of nicotine.

The formation of an intraoral formulation that contains an adequate amount of nicotine (or salt thereof) together with a pH regulating agent is difficult. We have found that, during the process of forming the hardened porous chemically bonded ceramic system, a significant proportion of the nicotine can be lost. Without wishing to be bound by theory, it is believed that the primary mechanism for loss is evaporation.

We have surprisingly found that this evaporative loss can be greatly reduced or essentially completely eliminated by allowing the chemically bonded ceramic system to harden and set without any pH regulating agent being present. Thus, in a second aspect of the invention, there is provided a method of forming an intraoral formulation comprising a solid, porous chemically bonded ceramic system, nicotine or a salt thereof, and a pH regulating agent, wherein the method comprises forming the chemically bonded ceramic system in the absence of said pH regulating agent and in the presence of nicotine or a salt thereof, and wherein the chemically bonded ceramic system contains calcium. These methods are hereinafter referred to as "methods of the invention". All embodiments and preferences disclosed herein in connection with the formulations of the invention are also disclosed in connection with the methods of the invention.

The nicotine (or salt thereof) is present during the hardening phase, i.e. the nicotine is intermixed with the precursor materials for the chemically bonded ceramic system, with the result that good control of the nicotine loading can be achieved. Thus, in the method of the invention, the chemically bonded ceramic system is formed in the presence of nicotine or a salt thereof. The pH regulating agent may then be introduced after the chemically bonded ceramic system has hardened without evaporative loss of nicotine occurring.

As is hereinbefore described, chemically bonded ceramic systems may alternatively be loaded with the nicotine or salt thereof after the chemically bonded ceramic mass has been formed, e.g. by soaking the ceramic material in a liquid containing the nicotine or its salt, or through any other method which facilitates the drawing up of that substance into the pores of the ceramic material via capillary forces (including spraying, brushing, rolling, dip coating, powder coating, misting or vacuum enhanced loading).

For the avoidance of doubt, it is not essential for all of the nicotine in the formulation to be located within the pores of the chemically bonded ceramic system. Some of the nicotine in the intraoral formulation may be located outside of the pores. For example, where the intraoral formulation is provided in the form a nicotine pouch containing the powdered formulation, a portion of the nicotine (preferably a powdered salt of nicotine) may be mixed with powdered chemically bonded ceramic system (which may itself comprise pores containing an amount of nicotine or salt thereof) prior to being placed in the pouch. Said mixing may be achieved by spraying a nicotine solution onto particles of the nicotine-loaded chemically bonded ceramic system, and then allowing that mixture to dry prior to being placed in the pouch. In such embodiments, the amount of nicotine (or salt thereof) that is added to the hardened, powdered chemically bonded ceramic system is from 1 to 50%, from 5 to 40% or from 10 to 30% by weight of the total amount of nicotine (or salt thereof) in the intraoral product.

In a further example, where the intraoral formulation is provided in the form of a nicotine pouch containing either the powdered formulation or a combination of a solid, porous chemically bonded ceramic system and nicotine (or a salt thereof), a portion of the nicotine may be associated with the material that forms the pouch, i.e. a portion of the nicotine is not located within cavity of the bag (as is described elsewhere herein) but is instead bound to or incorporated within the walls of the bag itself. This may be achieved e.g. by soaking the pouch material in a solution containing nicotine (as base or dissolved nicotine salt) and then allowing the solvent (e.g. water or another appropriate solvent) to evaporate leaving nicotine or a salt thereof incorporated into the pouch material. In embodiments in which the intraoral formulation is provided in the form an intraoral product that is a nicotine pouch and a portion of the nicotine in said intraoral product is associated with the material that forms the pouch, the amount of nicotine (or salt thereof) associated with the material may be from 0.1 to 30%, from 0.5 to 20% or from 1 to 10% by weight of the total amount of nicotine (or salt thereof) in the intraoral product.

References herein to methods of the invention in which the chemically bonded ceramic system is formed in the presence of nicotine or a salt thereof include methods in which the chemically bonded ceramic system is formed in the presence of only a portion (but not the totality) of the nicotine in the final intraoral product. In this way, the pores of the hardened chemically bonded ceramic system contain up to 99% (such as up to 95%, up to 90%, up to 80%, or up to 70%) by weight of the total amount of nicotine or salt thereof in the final intraoral product.

Typically, once a hardened mass of chemically bonded ceramic (e.g. a hardened mass of chemically bonded ceramic system which has been loaded with nicotine or a salt thereof) is formed, it is then ground to form a powder which is subsequently mixed with the pH regulating agent. The grinding process may also be performed in the presence of the pH regulating agent to ensure efficient mixing.

As the pH regulating agent is not present during the formation of the chemically bonded ceramic system, the pH regulating agent does not become incorporated into the pores of the chemically bonded ceramic system. Ingress of a small amount of the pH regulating agent into the pores of the chemically bonded ceramic system may occur when the two components are brought together or during subsequent processing, but only minimal ingress will occur in this way. Therefore, in one embodiment, the pH regulating agent is predominantly located outside of the pores of the chemically bonded ceramic system. By this, we mean that at least 90% (e.g. at least 95%, preferably at least 98%) by weight of the pH regulating agent is located outside of the pores of the chemically bonded ceramic system.

The term "pH regulating agent" refers to any substance capable of altering the pH of normal saliva. The pH of normal saliva is around 6.8. The pKa values of nicotine are about 3.2 and 8.02 (Tomar S. L. et al., Tobacco Control 1997;6:219-225). Absorption of nicotine from the oral cavity, i.e. transmucosal uptake, to the systemic circulation is dependent on the local pH of the saliva inside and close to the product in use. Nicotine will predominantly be absorbed through the mucosa in the non-protonated form. Therefore, it is preferable to provide a local pH which results in a high fraction of the nicotine being non-protonated. For this reason, alkaline conditions enhance transport of nicotine across the oral mucosa and into the bloodstream, and so it is preferred that the pH regulating agent is an alkaline agent, i.e. a substance capable of increasing the pH of saliva above normal. Alkaline buffering agents are also contemplated in this context. The term "alkaline buffering agent" may be used interchangeably with "alkaline buffer" and refers to agents for obtaining a buffer solution with an alkaline pH. Preferred alkaline agents are buffering agents selected from the group consisting of acetates, glycinates, phosphates, glycerophosphates, citrates such as citrates of alkaline metals, carbonates (including hydrogen carbonates), and borates, or mixtures thereof. In addition, strong bases may be used such as sodium hydroxide, potassium hydroxide, and mixtures thereof. In particular embodiments, a carbonate (including a hydrogen carbonate) or a phosphate (including a triphosphate) compound may be used as the pH regulating agent. Such compounds may be formed as salts with any suitable cation, such as sodium, potassium, calcium or magnesium. Particular pH regulating agents that may be mentioned include sodium carbonate, sodium bicarbonate, trisodium phosphate, and combinations thereof.

Further examples of pH regulating agents that may be used include calcium aluminates, calcium silicates, calcium phosphates and calcium carbonates, as well as chemically bonded ceramic systems made from any of these calcium salts (as described elsewhere herein), and mixtures thereof. For the avoidance of doubt, it is not essential that any pores in said chemically bonded ceramic systems contain nicotine or a salt thereof. When a chemically bonded ceramic system made from any of the calcium salts mentioned here is used as a pH regulating agent, that system is formed (e.g. through hardening a hydrated mass of the calcium salt) before it is added to the other components of the formulation of the invention. The pH regulating agent in such examples may be referred to as a "second" chemically bonded ceramic system. The formulations of the invention already contain a chemically bonded ceramic system (formed in the absence of the pH regulating agent), which may therefore be referred to as a "first" chemically bonded ceramic system.

In a particular example of such formulations of the invention, the second chemically bonded ceramic system (i.e. the pH regulating agent) may be formed from calcium aluminate, and the first chemically bonded ceramic system may be formed from calcium sulphate in the absence of the pH regulating agent. Once the first chemically bonded ceramic system is formed, it may be loaded with nicotine or a salt thereof through methods such as soaking the first chemically bonded ceramic system. However, the first chemically bonded ceramic system is preferably formed in the presence of the nicotine of salt thereof.

The amount of pH regulating agent present in the formulation is typically chosen to be sufficient to raise the pH of the saliva inside and close to the product in use to at least 8. In an embodiment of the invention, the formulation comprises said pH regulating agent in an amount of at least 0.1%, particularly at least 0.5%, by weight of the formulation. For example, the formulation may comprise said pH regulating agent in an amount of from 0.1% to 50% by weight of the formulation. Such weights are particularly suited to formulations in which the pH regulating agent is a sodium carbonate, i.e. when the pH regulating agent is sodium carbonate (Na₂CO₃), sodium bicarbonate (NaHCO₃) or a mixture thereof.

In some embodiments, the pH regulating agent is a buffering agent that comprises sodium carbonate and sodium bicarbonate, e.g. in a weight-ratio between 5:1 and 2.5:1, preferably in a weight-ratio between 4.1:1 and 3.5:1. A particular buffering agent that may be mentioned is the sodium carbonate - sodium bicarbonate buffer system.

### Extended Flavour Release

The solid, porous chemically bonded ceramic systems disclosed herein in respect of the first aspect of the invention have also been found to surprisingly effective at providing extended release of flavours in the mouth. As is shown in the examples, dry powders containing such ceramic systems in which one or more flavours was loaded into pores in the ceramic were surprisingly able to release the flavour at a sufficient rate and for a sufficient period to give a meaningful sensory experience for the user for substantially longer than was achieved by commercially available formulations. In some cases, the flavours were sustained for almost twenty times as long using the formulations of the invention when compared to commercial products.

Therefore, we also disclose a combination of a solid, porous chemically bonded ceramic system as disclosed herein and a flavour. The flavour is incorporated into pores in the chemically bonded ceramic system using any of the methods described above with respect to nicotine.

In one example, the chemically bonded ceramic systems used in the formulation of the invention is loaded with the flavour (or flavours) by soaking the ceramic material in a liquid containing the flavour, or through any other method which facilitates the drawing up of that flavour into the pores of the ceramic material via capillary forces (including spraying, brushing, rolling, dip coating, powder coating, misting or vacuum enhanced loading). Some flavours may, for example, by supplied as a liquid product, e.g. as a solution, suspension, or emulsion. Such liquid products may be brought into contact with the chemically bonded ceramic system through simple mixing of the two components in order for the flavour to become incorporated into pores in the ceramic. As is shown in the examples, the ceramic pores may also contain nicotine prior to the introduction of the flavour, and the product is still capable of providing extended release of both the nicotine and the flavour.

In another example, the flavour is co-formedly interspersed within pores in the chemically bonded ceramic system. For example, the precursors for the chemically bonded ceramic system may be mixed with flavour (or flavours) prior to the hardening process taking place. The flavour is then present (optionally together with the nicotine or salt thereof) at the moment when pore formation occurs with the result that the flavour becomes located within pores of the chemically bonded ceramic system.

Examples of flavours include sweeteners and flavours (or "flavourings") described elsewhere herein. For the avoidance of doubt, this includes sugars such as sucrose, glucose, dextrose, maltose and/or fructose, sugar alcohols such as mannitol, xylitol, sorbitol, maltitol and/or isomalt, or artificial sweeteners such as sucralose, cyclamates, aspartame, acesulfame and/or saccharin.

Other flavourings that may be used, particularly in nicotine pouches, include menthol, peppermint, wintergreen, sweet mint, spearmint, vanillin, chocolate, black cherry, coffee, cinnamon, clove, tobacco, citrus, fruit flavour and mixtures thereof. In one embodiment, the flavouring is not tobacco or nicotine. Such flavours may be present in the combination in an amount of at least 0.1%, and preferably not more than 50%, by weight of the combination of a solid, porous chemically bonded ceramic system and flavour (or flavours).

A flavour may be used in combination with any of the chemically bonded ceramic systems described elsewhere herein. For the avoidance of doubt, this includes any chemically bonded ceramic system formed from a calcium phosphate, a calcium sulphate, a calcium silicate, a calcium aluminate, a magnesium carbonate or a combination thereof. Preferred chemical compositions include those based on chemically bonded ceramic systems, which following hydration of one or more appropriate precursor substances consume a controlled amount of water to form a network. Precursor substances that may be mentioned in connection with said porous chemically bonded ceramic systems include CaOAl₂O₃, (CaO)₁₂(Al₂O₃)₇, (CaO)₃(Al₂O₃), (CaO)(Al₂O₃)₂, (CaO)₃(SiO₂), (CaO)₂(SiO₂), and, particularly, alpha-tricalcium phosphate, tetracalcium phosphate (Ca₄(PO₄)₂O), and calcium sulphate (e.g. calcium sulphate hemihydrate).

Combinations of a solid, porous chemically bonded ceramic system as disclosed herein and a flavour may be used in the manufacture of intraoral products, e.g. of the sort described herein, as well as food products (such as chewing gum, sweets, breath mints and the like), and health products that are intended to be held in the mouth for an extended period (e.g. more than 5 minutes). In one example, they may be used in the manufacture of nicotine pouches, e.g. by adding the combination to nicotine (or a salt thereof) before loading into a pouch according to any of the methods disclosed here. A combination of a solid, porous chemically bonded ceramic system as disclosed herein and a flavour may be advantageously provided for this or any other use as a powder, as the combination can be supplied as a powder that is easy to handle. Such combinations may be stored in any suitable container, e.g. an airtight container. They may also be supplied in any quantity, e.g. from 1g to 100 kg, for the purposes of manufacture.

In a further example, there is provided a pouch (e.g. a non-woven fabric pouch) containing a combination of a solid, porous chemically bonded ceramic system as disclosed herein and a flavour.

### Intraoral Formulations

The formulations of the invention are intraoral formulations that are intended to release nicotine (or a salt thereof) upon exposure to moisture in the mouth. The formulations are capable of both immediate and sustained release, but are particularly suited for products intended for slow and/or sustained release.

Intraoral formulations that are capable of providing a sustained release of nicotine allow the user to obtain a long-lasting sensory experience using a minimal number of formulations per day. Where the formulation is intended to be used as part of a therapeutic method of treating nicotine dependence, this characteristic improves patient compliance and minimises interference with the individual's lifestyle.

The term "sustained-release" is employed herein synonymously with the term "controlled-release", and will be understood by the skilled person to include formulations that provide, and/or are adapted to provide, for a "sustained", a "prolonged" and/or an "extended" release of nicotine (in which nicotine is released at a sufficiently retarded rate to produce a therapeutic response or give a pleasurable experience over an extended period of time compared to pouch formulations currently on the market).

The formulations of the invention are capable of achieving a nearly constant rate of release over an interval of from about 10 minutes to about 1 hour, potentially longer. In one embodiment, the formulations of the invention are capable of achieving a nearly constant rate of release over an interval of from about 10 to 30 minutes. What is meant by this is that nicotine release from the formulation continually occurs (at a non-zero rate) over the specified time interval. Constant release may further be defined as a composition being capable of maintaining a steady state concentration in a body fluid not deviating more than about 20% (e.g. about 10%) from the mean value during the dose interval.

The formulations of the invention, when held in the mouth, are advantageously capable of releasing a high proportion of the material held in the pores. By this we mean that, when the formulation is held in the mouth for an extended period of time (e.g. at least 10 minutes or preferably at least 20 minutes), at least 75% (e.g. at least 80%, at least 85% or at least 90%) of the nicotine stored in the pores of the chemically bonded ceramic system is released from said pores and made available to the user. This characteristic of near-complete release allows the user to know more accurately how much nicotine is taken into the body, which may be advantageous when dosing a patient for therapeutic purposes. When the formulations of the invention are used in nicotine pouches, such as those described herein where nicotine is held in pores in a chemically bonded ceramic system, once the nicotine (or salt thereof) has been released from those pores it is immediately able to escape the pouch and be taken up by the user.

In addition, the formulations of the invention are capable of achieving essentially complete release in a timeframe suitable for therapeutic use. As is demonstrated in the Examples, the formulations of the invention are capable of releasing almost all of the releasable nicotine in around 20 minutes when held in the mouth. Users of nicotine pouches typically hold an individual pouch in their mouth for a period of from five minutes to one hour whilst the nicotine is released, with 20 to 30 minutes being the most common timeframe. This characteristic helps to minimise wastage of unused nicotine when a product has been consumed, and makes the formulations of the invention, and pouches containing said formulations, particularly well-suited to use in therapeutic treatment, such as nicotine replacement therapy or aiding smoking cessation.

The dry formulations of the invention have also been found to provide rapid release of the water soluble components. Studies involving human volunteers found that the onset of sensations associated with flavours was more rapid for the dry formulations of the invention compared with dry commercial formulations. The time required for onset of nicotine sensation for the dry formulations of the invention was equal to or less than that for dry commercial formulations. The formulations of the invention are therefore capable of generating a rapid sensory experience for the user.

The total amount of nicotine that is delivered by each formulation is preferably from about 0.5 mg to about 15 mg, e.g., from about 1 mg to about 10 mg. For example, the formulation may deliver from about 1 mg to about 8 mg, from about 1.5 mg to about 7.5 mg, from about 2 mg to about 5 mg, from about 2.5 mg to about 5 mg, from about 3 to about 10 mg, from about 3 to about 7.5 mg or from about 3 mg to about 5 mg. In a further example, the formulation may contain about 1.5 mg, about 2 mg, about 2.5 mg, about 3 mg, about 3.5 mg, about 4 mg, about 5 mg or about 6 mg, as calculated as free nicotine base. In particular the amount of nicotine is 2 mg, 3 mg, 4 mg or 6 mg. The above values refer to the amount of nicotine in the free base form that can be delivered from each formulation, irrespective of whether the nicotine in the formulation is provided as free base, a salt or in any other form.

The formulations of the invention are suitable for use in conventionally sizes nicotine pouches that are intended to be used by holding the pouch in the mouth under the lip. Typically, such pouches are rectangular and have a length of about 25 to about 35 mm, and a width of about 10 to about 15 mm.

The formulations of the invention may also provide a relatively concentrated form of nicotine, thus enabling the manufacture of small pouches that still contain a conventional amount of nicotine and have advantageous release properties as described herein. Formulations of the invention may contain at least 50 mg (such as least 60 mg, at least 70 mg or at least 80 mg) nicotine (calculated as free base irrespective of the form in which it is present) per gram of powder. By way of an example, 12 mg of nicotine (calculated as free base) may be incorporated into a chemically bonded ceramic system to give a powder with a total mass of no more than 0.15 g. In this context, small pouches may have no dimension larger than 20 mm, preferably no dimension larger than 15 mm. Typically, small pouches are rectangular and have a length of about 10 to about 15 mm, and a width of about 8 to about 10 mm.

The amount of nicotine contained within the intraoral formulation prior to use may exceed the amount of nicotine that is intended to be delivered to the user. This is because a proportion of the nicotine may be trapped within the chemically bonded ceramic system in such a way that complete release of the nicotine is not possible within the likely time period of use. However, preferably the formulation is capable of releasing substantially all of the nicotine or salt thereof upon contact with an aqueous liquid (e.g. saliva). By this, we mean that, upon contact with an aqueous liquid, the formulation is capable of releasing at least 80% (e.g. at least 90%) by weight of the nicotine or salt thereof.

The intraoral formulations may be supplied to users in the form of intraoral products that include nicotine pouches (similar to snus) and tablets. The term "nicotine pouch" as used herein refers to a pouch or bag that is fully or partially loaded with a combination of a solid, porous chemically bonded ceramic system and nicotine (or a salt thereof). In one embodiment, the pouch also contains a pH regulating agent; in such cases the pouch or bag may be said to be fully or partially loaded with a formulation of the invention. In another embodiment, pH regulating agent is incorporated into (e.g. bound to) the material that forms the pouch or bag. Nicotine pouches which comprise a pH regulating agent both within the cavity of the pouch or bag and incorporated into the material that forms said pouch or bag are also contemplated.

The formulations of the invention are particularly suited for transmucosal administration whereby the intraoral product (e.g. in the form of a tablet, wafer, lozenge or similar product) containing the formulation is placed in contact with the lip, gum or cheek for an extended period (several minutes) while the nicotine is released. The intraoral products (e.g. pouches and tablets containing the formulation of the invention) may be supplied to the end user in bulk. That is, the present invention also relates to a package containing a plurality of intraoral products (e.g. at least two pouches or tablets).

A nicotine pouch containing the formulation of the invention may be described as a tobacco-free version of snus. In such systems, the pouch or bag contains a specific amount of the formulation, and thereby a specific amount of nicotine, but typically does not include any tobacco dust, leaf or stem.

An embodiment of the present invention concerns a nicotine pouch comprising a permeable, sealed bag containing a solid, porous chemically bonded ceramic system, nicotine or a salt thereof, and a pH regulating agent, wherein the chemically bonded ceramic system is formed in the absence of said pH regulating agent. The bag is typically made of a permeable material that encloses a cavity. The powdered intraoral formulation is held within the cavity, but soluble components of the formulation are able to pass through the bag material when the bag is exposed to water (e.g. saliva). Suitable materials for nicotine pouches are known to the skilled person, and include paper of the sort used in tea bags, filter paper, and the like. Other materials include heat-sealable non-woven cellulose, such as long fiber paper, cotton and silk.

The formulation of the invention also provides stable storage of the nicotine (or salt thereof) prior to use. The formulation of the invention is preferably stored in an airtight container, such as a tin or bag, prior to use, and it may be stored in this way for several weeks or months (e.g. up to at least one year) without significant loss of the nicotine. Suitable storage containers are known to the skilled person and include any conventional closable container. These storage containers may provide a convenient and portable system capable of holding multiple pouches or tablets. A further aspect of the invention therefore relates to a closable container comprising one or more, and preferably a plurality of, intraoral products of the invention (e.g. nicotine pouches or sublingual and buccal tablets as described herein).

As is hereinbefore mentioned, the pH regulating agent is not present during the formation of the chemically bonded ceramic system, so the pH regulating agent does not become incorporated into the pores of the chemically bonded ceramic system. The pH regulating agent may therefore be incorporated into the final product through other methods, e.g. by mixing the pH regulating agent with the hardened chemically bonded ceramic system as is hereinbefore described. Alternatively, or additionally, the pH regulating agent may be associated with the bag material itself. For example, particles of the pH regulating agent (e.g. sodium carbonate and/or sodium bicarbonate) may be embedded within the permeable material of the bag so that the pH regulating agent is readily able to dissolve upon contact with saliva. Thus, in one embodiment, the bag comprises a cavity surrounded by a permeable material, and the pH regulating agent is associated with the permeable material. Other suitable methods of incorporating the pH regulating agent into the bag material would be known to the skilled person.

In an embodiment of the invention, the intraoral formulation is contained within a pouch, and the total weight of the loaded pouch is from 0.2 to 3 g, such as from 0.4 to 2 g. In another embodiment, the nicotine pouch has a weight and/or volume similar to commercially available portion snus products and nicotine pouches. In a further embodiment of the invention, the intraoral formulation is contained within a small pouch having a total weight of from 0.1 to 0.5 g, such as from 0.1 to 0.4 g. Small pouches are discussed elsewhere herein and the utility of such low weight products is possible thanks to the fact that the chemically bonded ceramic systems referred to herein are capable of providing a very compact and stable storage of nicotine whilst maintaining a suitable release profile.

Nicotine pouches may be manufactured using methods known to those skilled in the art, particularly those methods used for the manufacture of snus and commercially available nicotine pouches (such as ZYN^{®}). For example, the powdered contents for the pouch may be made using the methods described herein or using conventional methods known in the art, and the powder may then be then loaded into sealable bags, e.g. heat-sealable bags. Such bags should be water insoluble and permeable to saliva. Suitable materials for nicotine pouches are described hereinbefore, and are also known to the skilled person, for example from US patent no. 9,161,908. Heat-sealable non-woven cellulose, such as long fiber paper, offers a particularly suitable material for use in nicotine pouches. Once a prescribed amount of the powder is filled into the pouch, it is maintained in the pouch by sealing. Uptake of nicotine in the mouth may be facilitated by incorporating a bioadhesion and/or mucoadhesion promoting agent into the nicotine pouch. The bioadhesion and/or mucoadhesion promoting agent may be provided in cavity of the bag. Alternatively, or additionally, that agent may be incorporated into or combined with the bag material.

Tablet-based intraoral products that may be mentioned include sublingual tablets, buccal tablets, wafers and lozenges. In this form, the intraoral product containing the formulation of the invention is intended to be placed under tongue, under the lip, against the gum, or against the cheek, and the nicotine is absorbed through the surrounding mucous membranes. References to "sublingual tablets" elsewhere herein include references to buccal tablets except where indicated otherwise. Adhesion to the interior surface of the mouth may be facilitated by incorporating a bioadhesion and/or mucoadhesion promoting agent into the sublingual tablet, wafer or lozenge.

The bioadhesion and/or mucoadhesion promoting agent is effective in making the tablet or pouch adhere to the oral mucosa and may, in addition, possess properties to swell and expand in contact with water and thus make a tablet disintegrate when wetted with saliva.

The expression "mucoadhesion" is meant to denote an adhesion to mucous membranes which are covered by mucus, such as those in the oral cavity, while the expression "bioadhesion" is meant to denote an adhesion to biological surfaces more in general, including mucous membranes which are not covered by mucus. These expressions generally overlap as definitions, and may usually be used interchangeably, although the expression "bioadhesive" has a somewhat wider scope. In the present specification and claims, the two expressions serve the same purpose as regards the objects of the invention, and this has been expressed by the use of the common term "bio/mucoadhesion". Suitably the tablet contains from 0.1 up to 25 weight percent of bio/mucoadhesion promoting compound, based on the total weight of the tablet.

It is preferred that the bio/mucoadhesion promoting agent is a polymeric substance, preferably a substance with an average molecular weight above 5,000 Daltons (weight average). A variety of polymers known in the art can be used as bio/mucoadhesion promoting agents. Examples of such bio/mucoadhesion promoting agents include cellulose derivatives such as hydroxypropylmethyl cellulose (HPMC), hydroxyethyl cellulose (HEC), hydroxypropyl cellulose (HPC), methyl cellulose, ethyl hydroxyethyl cellulose, carboxymethyl cellulose, modified cellulose gum and sodium carboxymethyl cellulose (NaCMC); starch derivatives such as moderately cross-linked starch, modified starch and sodium starch glycolate; acrylic polymers such as carbomer and its derivatives (Polycarbophyl^{®}, Carbopol^{®}), etc.); polyvinylpyrrolidone; polyethylene oxide (PEO); chitosan (poly- (D-glucosamine)); natural polymers such as gelatin, sodium alginate, and pectin; scleroglucan; xanthan gum; guar gum; poly co-(methylvinyl ether/maleic anhydride); microcrystalline cellulose (Avicel^{®}); and croscarmellose (e.g. crosscarmellose sodium). Such polymers may be crosslinked. Combinations of two or more bio/mucoadhesive polymers can also be used. Substances like HPMC that can produce a smooth surface for the finished tablet whilst still enabling the tablet to stick to oral mucosa are particularly advantageous.

In an embodiment of the invention, the intraoral formulation is provided in the form of a tablet having a total weight of from 25 to 200 mg, such as from 50 to 150 mg.

Tablets (e.g. sublingual tablets) may be manufactured using methods known to those skilled in the art, such as dry powder blending, granulation and tablet pressing. Tablets may also be made by mixing the dry ingredients, including the precursor(s) for the chemically bonded ceramic system, adding water, wet mixing and then casting.

The final tablet may, for example, be a buccal tablet that does not fully dissolve in the mouth but which needs to be physically removed (e.g. swallowed) once used. The tablet may alternatively be a dissolving tablet in which all components are dissolved and either absorbed or swallowed.

Nicotine pouches and tablets may also contain one or more other ingredients selected from the group consisting of a filler (typically a food grade filler), water, salt, and flavouring. It is not essential that any of these other ingredients is incorporated into the pores of the chemically bonded ceramic system, however any such ingredients that are incorporated in this way and are water soluble will be expected to have a similar release profile to the nicotine contained within those pores.

Fillers that increase saliva production may also advantageously be used in the products described herein. Such substances, sometimes known as sialogogues, are known in the art and include salt (NaCl), sweeteners (e.g. xylitol)_and acidic substances (such as malic acid and ascorbic acid). The nicotine in the product of the invention is able to dissolve in saliva and is then be absorbed through the oral mucosa. Flavourings in the products are also transported by saliva to taste buds in the oral cavity. The incorporation of a substance that increases saliva production improves the experience for the user by ensuring that the sensations arising from the nicotine and flavourings in the product can occur more quickly.

Other fillers that may be used in nicotine pouches include rubber arabicum, microcrystalline cellulose, maltitol. Yet more fillers that may be mentioned include inert inorganic fillers such as alumina, zirconia, and glass.

Fillers that may be used in sublingual tablets (and wafers, lozenges, etc.) include conventional fillers known to the skilled person in the context of pharmaceutical formulations, particularly oral tablets and capsules. Appropriate materials are well known to the person skilled in the art; see, for instance: Dosage Forms: Tablets. Volume 1, 2nd Edition, Lieberman H A et al. New York and Basel 1989, p. 354-356, and literature cited therein.

Fillers are typically present in the intraoral product in an amount ranging from about 10% to about 90% by weight of the intraoral product.

Controlled release agents may also be incorporated into the formulation. Such agents slow the rate of release of nicotine from the product and thereby extend the duration of the sensory experience for the user. It is preferred that the controlled-release agent is a material that is capable of providing a sustained-release, a delayed-release or both.

In this respect, it is preferred that the controlled-release agent is a polymer. Examples of polymers that may be employed as controlled-release agents include, without limitation: alkylcellulose polymers (e.g. ethylcellulose polymers), and acrylic polymers (e.g. acrylic acid and methacrylic acid copolymers, methacrylic acid copolymers, methyl methacrylate copolymers, ethoxyethyl methacrylates, cyanoethyl methacrylate, methyl methacrylate, copolymers, methacrylic acid copolymers, methyl methacrylate copolymers, methyl methacrylate copolymers, methacrylate copolymers, methacrylic acid copolymer, aminoalkyl methacrylate copolymer, methacrylic acid copolymers, methyl methacrylate copolymers, poly(acrylic acid), poly(methacrylic acid, methacrylic acid alkylamid copolymer, poly(methyl methacryate), poly(methacrylic acid) (anhydride), methyl methacrylate, polymethacrylate, methyl methacrylate copolymer, poly(methyl methacrylate), poly(methyl methacrylate) copolymer, polyacryamide, aminoalkyl methacrylate copolymer, poly(methacrylic acid anhydride), and glycidyl methacrylate copolymers). The polymer may also be a mixture of polymers. Typically, the molecular weight (weight average and/or number average) of the polymer is 1,000 to 10,000,000, 10,000 to 1,000,000, preferably 50,000 to 500,000 g/mol, as measured by gel permeation chromatography.

Preferred polymers include the alkyl cellulose polymers and acrylic polymers described herein.

In order to improve the sensory properties of the formulation of the invention, one or more sweeteners or texture improvers may be added. These substances include sugars such as sucrose, glucose, dextrose, maltose and/or fructose, sugar alcohols such as mannitol, xylitol, sorbitol, maltitol and/or isomalt, or artificial sweeteners such as sucralose, cyclamates, aspartame, acesulfame and/or saccharin. Sweeteners are particular useful for masking the taste of the nicotine in the formulation.

Other flavourings that may be used, particularly in nicotine pouches, include menthol, peppermint, wintergreen, sweet mint, spearmint, vanillin, chocolate, black cherry, coffee, cinnamon, clove, tobacco, citrus, fruit flavour and mixtures thereof. In one embodiment, the flavouring is not tobacco. The formulations of the invention may be used with any flavour or combination of flavours. Such flavour may be present in an amount of at least 0.1%, and preferably not more than 5%, by weight of the nicotine pouch or tablet.

Substances that give a cooling mouthfeel, such as sugar alcohols (e.g. erythritol), may be used in the intraoral products disclosed herein.

The nicotine pouches described herein may also contain a moist intraoral formulation. A moist intraoral formulation is an intraoral formulation of the invention which additionally contains a component which is either liquid under ambient conditions or is a low-melting solid. Suitable components are known in the art, and include glycerol, propylene glycol ("PG", E1520), polyethylene glycol ("PEG") and sodium alginate (E401). The addition of a relatively small amount of such components advantageously binds the smallest particles together to minimise the formation of fine dust. Fine dust can travel through, or block pores in, the sort of non-woven fabrics typically used in the manufacture of nicotine pouches. Typically, the liquid or low-melting solid is added after the powder has been formed (e.g. by crushing a larger solid mass), and optionally after coarse particles of the chemically bonded ceramic system have been removed. The skilled person would be able to determine the amount of liquid or low-melting solid that needs to be added to the formulation of the invention. A suitable amount may range from 0.1% to 10% (such as 2 to 5%) by weight of the formulation. It is preferred that the amount is sufficiently low to yield a powdery material that is easily manufactured, handled, transported, divided and/or processed.

Moist pouches, i.e. pouches that contain a moist intraoral formulation, generally feel more comfortable in the mouth, and typically provide for quicker initial release of nicotine compared to dry pouches. However, we have found that moist intraoral formulations of the present invention also have surprising sustained release characteristics, similar to the dry formulations. That is, the moist formulations are capable of releasing nicotine (or a salt thereof), as well as other flavours over a sustained period of time. The use of PG, or any other moistening agents of the sorts mentioned hereinabove could also advantageously help prevent unwanted release of material from the pouch, e.g. by agglomerating the finer parts of the powder which may otherwise be able to traverse through the pores in the pouch membrane.

A moist intraoral formulation typically has the form of a dough or paste. Such formulations may be made by simple mixing of the liquid or low-melting component with a dry formulation of the invention.

Oral formulations of the invention may also contain one or more preservatives. Substances that may be used as preservatives include sodium benzoate and potassium sorbate as well as other substances known in the art to have similar functionality. Preservatives may be particularly suitable for use in moist intraoral formulations, e.g. those which contain glycerol and/or propylene glycol.

Particular intraoral formulations that may be mentioned include intraoral formulations comprising:
a solid, porous chemically bonded ceramic system formed from a material selected from the group consisting of alpha-tricalcium phosphate, tetracalcium phosphate, calcium sulphate and combinations thereof;
a pH regulating agent that comprises a sodium carbonate (i.e. sodium bicarbonate and/or sodium carbonate); and
nicotine or a salt thereof;
wherein the chemically bonded ceramic system contains calcium and is formed in the absence of said pH regulating agent, and wherein the chemically bonded ceramic system is formed in the presence of nicotine or a salt thereof such that said nicotine or salt thereof is located in pores in the chemically bonded ceramic system. Said intraoral formulations may be advantageously provided in the form of a sublingual or buccal tablet (such as a tablet containing a bioadhesion and/or mucoadhesion promoting agent) or nicotine pouch (i.e. a permeable, sealed bag containing the solid, porous chemically bonded ceramic system, nicotine or a salt thereof, and pH regulating agent).

### Medical and Recreational Uses

The formulations of the invention have medical and/or recreational use.

The formulations may be placed in the mouth whereupon they are moistened through contact with saliva. The formulation may, for example, be placed in the mouth in contact with the lip, gum or cheek, and left there for an extended period. Typically, the formulation is retained in the mouth for a period of from five minutes to one hour. Longer durations are possible if the formulation is tailored to provide slower sustained release of the nicotine, e.g. by including a controlled release agent such as hydroxypropyl methyl cellulose.

In a medical context, the formulations of the invention may be used in the treatment of nicotine dependence, (e.g. nicotine addiction) with a view to aiding an individual in reducing smoking or stopping altogether. The formulations of the invention may therefore be described as being useful in aiding smoking cessation. The formulations of the invention have several benefits that make them particularly suited to aiding smoking cessation. Firstly, they are capable of releasing almost all of the nicotine held in the formulation, and this in turn allows a user or a clinician to know accurately how much nicotine is being administered to the patient. Secondly, the formulations provide essentially complete release in a timeframe suitable for therapeutic use, such as within 20 minutes or within 30 minutes. This timeframe is consistent with the amount of time that commercially available nicotine pouches are typically held in the mouth when used. Wastage of nicotine is also minimised as there is very little unused nicotine once a product has been used.

In a further aspect of the invention there is provided a method of treating nicotine dependence (e.g. nicotine addiction) wherein the method involves administration of a formulation of the present invention to a person suffering from symptoms of nicotine dependence. Similarly, the formulations of the invention may be useful in a method of treating (e.g. alleviating) the symptoms of nicotine dependence (including nicotine addiction or nicotine withdrawal), or aiding smoking cessation. Such symptoms may include cravings for nicotine, anger/irritability, anxiety, depression, impatience, trouble sleeping, restlessness, hunger or weight gain, and/or difficulty concentrating. Said use may also be referred to as nicotine replacement therapy. The references herein to methods of treatment by therapy are to be interpreted as references to intraoral formulations of the present invention for use in those methods.

Nicotine may also be used to ameliorate symptoms associated with various diseases, including dementia, Alzheimer's disease, Parkinson's disease, Huntington's disease, and depression. Thus, in a yet further aspect of the invention there is provided a method of ameliorating symptoms associated with dementia, Alzheimer's disease, Parkinson's disease, Huntington's disease, and depression, wherein the method involves administering a formulation of the present invention to a person suffering from said symptoms.

Formulations the present invention are capable of releasing a pharmacologically effective amount of nicotine during normal use. By "pharmacologically effective amount", we refer to an amount of nicotine which is capable of conferring a desired therapeutic effect on a treated patient, whether administered alone or in combination with another active ingredient. Such an effect may be objective (i.e. measurable by some test or marker) or subjective (i.e. the subject gives an indication of, or feels, an effect).

More preferred formulations of the invention may be adapted (for example as described herein) to provide a sufficient dose of nicotine over the dosing interval (irrespective of the number of doses per unit time) to produce a desired therapeutic effect.

The amounts of active ingredients that may be employed in formulations of the invention may thus be determined by therapeutic standards, the physician, or the skilled person, in relation to what will be most suitable for an individual patient or the condition to be treated. This is likely to vary with the type and severity of the condition that is to be treated, as well as the age, weight, sex, renal function, hepatic function and response of the particular patient to be treated.

Suitable daily dosages of nicotine, both for medicinal and recreational purposes, may be from about 1 to about 100 mg/day. Conventional cigarettes typically contain between about 8 and 15 mg nicotine. In one embodiment, each nicotine pouch or tablet of the invention is capable of delivering from about 3 to about 15 mg nicotine. It is preferred that the products disclosed herein are capable of delivering an amount of nicotine that is at least equivalent to one cigarette. When the nicotine is supplied to the patient in the form of a tablet (e.g. sublingual or buccal tablets), then each tablet may contain from about 3 to about 15 mg nicotine, e.g. from about 8 mg to about 15 mg of nicotine.

The above-mentioned dosages are exemplary of the average case; there can, of course, be individual instances where higher or lower dosage ranges are merited, and such are within the scope of this invention.

Ingress of moisture into the pores of the chemically bonded ceramic system results in the release of the nicotine (or salt thereof) contained within. The rate and extent of nicotine absorption into the body via the oral mucosa is increased when the alkalinity of the oral environment at the site of absorption in the mouth is increased, and the pH regulating agent in the formulation facilitates this. In an embodiment of the invention, at least 50% by weight of the nicotine released from the formulation is absorbed through the oral mucosa.

Nicotine may be released alongside a sweetener and/or other flavours which help to mask the taste of the nicotine whilst ensuring that the intended benefits arising from the absorption of the nicotine are still achieved.

For the avoidance of doubt, by "treatment" we include the therapeutic treatment, as well as the symptomatic treatment, the prophylaxis, or the diagnosis, of the condition.

When used for recreational purposes, the formulations are capable of delivering a sufficient amount of nicotine to provide a pleasurable sensory experience for the user.

The formulations of the invention may also be capable of improving the oral health of the user. As is discussed hereinbefore, this is particularly the case for formulations in which the chemically bonded ceramic system contains calcium and is capable of releasing this mineral (e.g. in the form of solubilised calcium ions) into the saliva and optionally also increasing the pH of the saliva inside and close to the product in use through the release of hydroxide ions. Solubilised calcium aids with remineralisation of teeth and bone, and so can contribute to mineral growth on the surfaces of teeth. Intraoral formulations of this sort have potential utility in repairing dental enamel and strengthening teeth.

The formulations of the invention may have the advantage that they provide an extended release of nicotine (e.g. nicotine is released at a sufficiently retarded rate to produce a therapeutic response or give a pleasurable experience over an extended period of time compared to pouch formulations currently on the market). The formulations of the invention may also have the advantage that they may be more efficacious than, be less toxic than, be faster acting than, be more potent than, produce fewer side effects than, be more easily absorbed than, and/or have a better pharmacokinetic profile than, have improved bioavailability over, and/or have other useful pharmacological, physical, or chemical properties over, compositions known in the prior art.

The use of the chemically bonded ceramic systems described herein affords for the provision of products which provide acceptable levels of release of nicotine when placed in the mouth, while minimising the risk of exposure to the stored nicotine (or salt thereof) within, e.g. through leakage.

The chemically bonded ceramic systems are also easily manufactured without the need for high temperature sintering, and therefore additional elements such as flavours, fillers and the like can be incorporated into the carrier to aid in the achieving the desired sensory experience. The ability to incorporate the nicotine (or salt thereof) into the carrier as the network structure is formed also allows for greater control over the amount of nicotine that is ultimately delivered to the user.

Wherever the word "about" is employed herein in the context of dimensions (e.g. values, temperatures, pressures (exerted forces), relative humidities, sizes and weights, particle or grain sizes, pore sizes, timeframes etc.), amounts (e.g. relative amounts (e.g. numbers or percentages) of particles, individual constituents in a composition or a component of a composition and absolute amounts, such as doses of nicotine, numbers of particles, etc.), deviations (from constants, degrees of degradation, etc.) it will be appreciated that such variables are approximate and as such may vary by ± 10%, for example ± 5% and preferably ± 2% (e.g. ± 1%) from the numbers specified herein.

The invention is illustrated by the following examples in which:
Figure 1 shows the release profiles for nicotine loaded powders made in the presence of sodium bicarbonate (triangles) and absence of sodium bicarbonate (circles);
Figure 2 shows the impact of different drying conditions and the presence of absence of sodium bicarbonate on the amount of nicotine that can be release from moulded tablets;
Figures 3a and 3b show the nicotine release profiles for a powdered mixture with a particle size of 100-200 µm stored within ZYN^{®} pouches alongside commercial Zyn^{®} Mini Dry (particle size 100-200µm) with two time scales;
Figures 4a and 4b show nicotine release (Fig 4a) and pH measurements (Fig 4b) for three powdered mixtures according to the invention;
Figures 5a and 5b show nicotine release (Fig 5a) and pH measurements (Fig 5b) for moist pouches of the invention (containing propylene glycol) and dry pouches of the invention;
Figures 6a to 6d show nicotine release and pH results after storage of a dry formulation of the invention, a dry commercial product and a moist commercial product for up to 34 days under either 33% or 60% RH;
Figure 7 shows the nicotine release profiles for a mixture of an alkaline bioceramic pH regulating agent and calcium sulphate loaded with nicotine;
Figure 8 shows the pH profiles for a mixture of an alkaline bioceramic pH regulating agent and calcium sulphate loaded with nicotine;
Figure 9 shows the percentage nicotine released from various products during 20 mins of use.

### Examples

### Example 1 - Impact of sodium bicarbonate and sodium carbonate on nicotine during loading

### Materials

The nicotine salt used was a USP grade nicotine bitartrate dihydrate. The pH regulating agent used was standard food grade sodium bicarbonate (referred to herein as E500). A BP, DAB grade calcium sulphate hemihydrate was used to form the chemically bonded ceramic system.

### Method

### (i) Without E500:

12.4954 g of BP, DAB grade calcium sulphate hemihydrate and 0.5252g of nicotine bitartrate dihydrate were dry mixed using a Turbula mixer at 46 rpm for 10 minutes.

As a next step, 10.0 g of water was added to form a paste by hand mixing using a spatula. The paste was cast into a rectangular shaped plate approximately 11 x 13 cm and 2 mm thick. The plate was left to harden at 35°C and ~95% humidity for approximately 1 hour whereafter the hardened paste was left to dry in room temperature for about 18 hours before crushing and sieving. The dried plates were crushed by hand using a spoon, pestle and mortar and sieved using a Retsch AS 200 Basic sieve shaker stack to obtain particle size fractions ranging from 100 µm to 500 µm.

### (ii) With E500:

0.5258g of nicotine bitartrate dihydrate and 0.5249 g of E500 was mixed with 10 mL of water. As a next step, the liquid was added to 12.5064 g of calcium sulphate hemihydrate BP, DAB grade to form a paste by hand mixing using a spatula. The paste was cast into a rectangular shaped plate approximately 11 x 13 cm and 2 mm thick. The plate was left to harden at 30°C and ~95% humidity overnight whereafter the hardened paste was left to dry in room temperature for about 18 hours before crushing and sieving. The dried plates were crushed by hand using a spoon, pestle and mortar and sieved using a Retsch AS 200 Basic sieve shaker stack to obtain particle size fractions.

### Analysis

All crushed powders were put in "Snuff" pouches commercially available for home making of wet snuff. The pouches are made out of non-woven cellulose and can be welded using gentle heat.

Extraction experiments were performed using a dissolution test set up according to USP 711 Apparatus 2 with mini vessels. In short, the samples were put in 100ml of phosphate buffer pH 6.8 at 37°C with the stirring paddles at 50rpm. 1 ml samples were then withdrawn for analysis at different timepoints. The amount of dissolved nicotine was determined using an HPLC system.

### Results and Conclusions

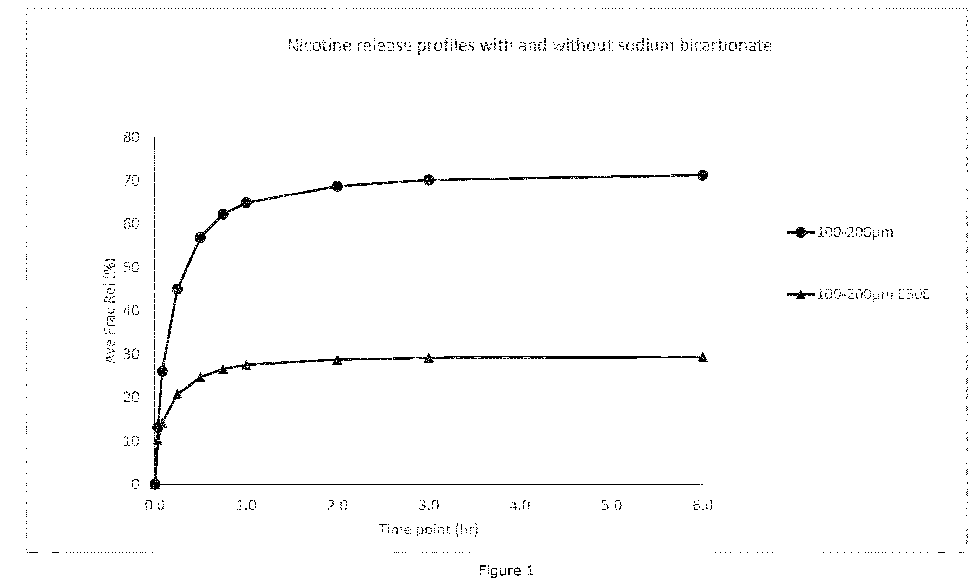

In the absence of E500 during preparation of the paste, the extractable amount of nicotine present in the powder was 70% of the amount of nicotine that was loaded. When E500 was present during loading, the extractable amount of nicotine present in the powder was 25-30% of the amount of nicotine that was loaded. Results are shown in Figure 1.

Addition of sodium bicarbonate during manufacture of the paste drastically decreased the amount of nicotine that was extractable from the samples.

Analogous experiments were also conducted in which a crushed powder was produced according to method (i) above, and then mixed with an amount of sodium bicarbonate corresponding to the amount used in method (ii). This mixture was also placed into "Snuff" pouches. Analysis of nicotine release was assessed using the method above and the release profile was found to be in line with that observed for the powder manufactured by method (i) and not subsequently mixed with sodium bicarbonate. This confirms that the sodium bicarbonate only influenced the nicotine release profile of the final powder when the sodium bicarbonate was present during the step of hardening the calcium sulphate hemihydrate aqueous paste.

Further analogous experiments have also been conducted using sodium carbonate in place of sodium bicarbonate. The results of these experiments showed that the addition of sodium carbonate to the mixture containing water, nicotine bitartrate dihydrate and calcium sulphate hemihydrate also lowered the nicotine content in the finished powder.

### Example 2

This experiment was conducted to further explore the mechanism behind the nicotine loss observed in Example 1.

### Method

Samples formed using BP and DAB grade calcium sulphate hemihydrate and nicotine in the form of USP grade nicotine bitartrate dihydrate were prepared according to the methods descried in Example 1. Half of the samples contained sodium bicarbonate and half did not.

In contrast to Example 1, the paste was moulded into tablets using empty tablet blisters and then hardened under different conditions described below (instead of casting a plate that is crushed and sieved).

Two sets of hardening conditions were used:
"Zip": the moulded paste was stored at room temperature ~24 °C in an airtight zip bag not much larger than the blister.

100% RH: the plate was stored at about 100% relative humidity and 30 °C for a period of time and then "dried" at room temperature before analysis.

### Analysis

The analysis was performed by extraction of the moulded tablets. Each tablet was put in a 50 mL Falcon tube with 15 mL of distilled water. The tubes were put on a shaker table for approximately 1 hour whereafter the extract was analysed with a UV-spectrophotometer system (VWR UV-3100PC) at a wavelength of 260 nm. Standard curves were obtained using defined solutions with nicotine base.

### Results

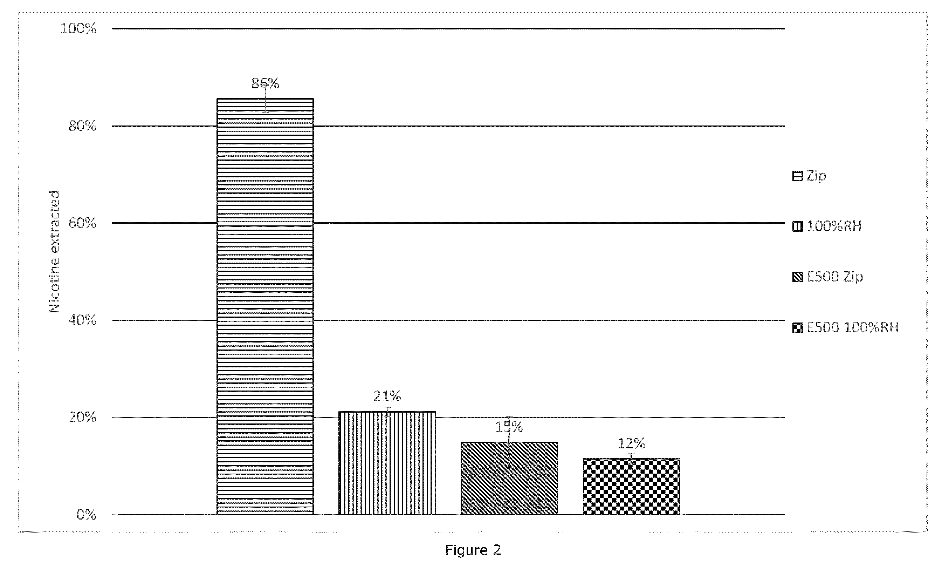

The results are shown in figure 2. Adding sodium bicarbonate (E500) to the formulation during manufacturing of the paste significantly decrease the amount of extractable nicotine. Letting the material set and harden in a moist and warm environment significantly decreased the amount of extractable nicotine.

The results show that the addition of the pH regulating agent should be made either by mixing the dry components after the chemically bonded ceramic system is hardened, crushed and sieved or incorporated into the formulation in some other fashion.

### Example 3 - Comparison with commercial products

### Method

Samples formed using BP and DAB grade calcium sulphate hemihydrate and nicotine in the form of USP grade nicotine bitartrate dihydrate were prepared and analysed according to the methods descried in Example 1. The dried plates were crushed by hand using a spoon, pestle and mortar and sieved using a Retsch AS 200 Basic sieve shaker stack to obtain particle size fractions. In this example only the 100 - 200 µm size fraction was used. The particles were put into "Zyn^{®}" pouches that had been emptied and cleaned. The pouches were then re-sealed using heat.

The commercial product tested was a dry well-known Swedish product, Zyn^{®} Mini Dry, purchased at a convenience store. For the Zyn^{®} product ten pouches were cut open and the powder inside was removed and sieved as in examples above. The 100 - 200 µm fraction was selected and put back into the pouches and sealed.

Analysis of nicotine release was performed as described in Example 1.

### Results

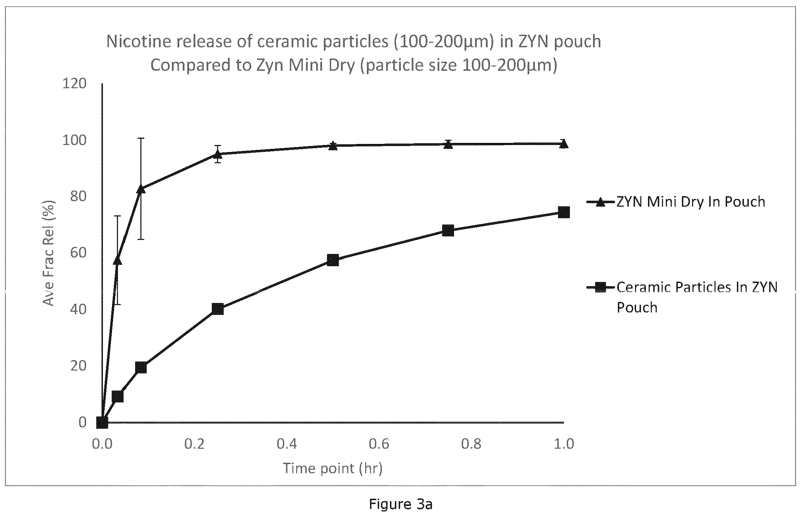

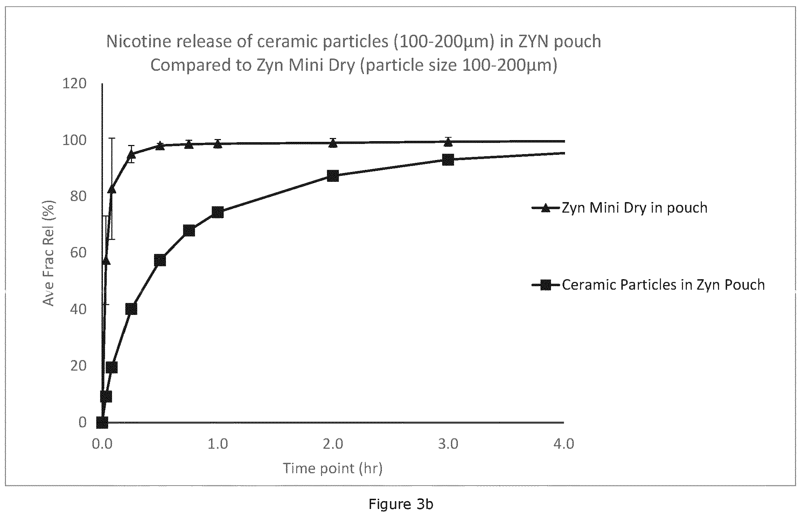

Incorporation of nicotine into a chemically bonded ceramic system formed from calcium sulphate was shown to result in a release of nicotine on the same level but with prolonged release compared to the commercial Zyn^{®} Mini Dry product. Results are shown in figures 3a and 3b. For comparison reasons, the scale on the Y-axis is normalised, meaning that for each curve the amount extracted at the last time point measured is set as 100%.

### Example 4 - pH comparison with commercial products

### Method

Samples using BP and DAB grade calcium sulphate hemihydrate (CaSH), nicotine in the form of USP grade nicotine bitartrate dihydrate were prepared according to the following:
23.1186 g CaSH was weighed onto a Al-foil sheet. 2.4886 g of nicotine bitartrate dihydrate was dissolved in 10 g of distilled water in a beaker. The nicotine - water solution was added to the powder and the beaker was rinsed with 8 g of distilled water that was added to the mix. The mixture was stirred by hand for about 1 minute and then spread out evenly on the sheet. The material was left to set and dry at room temperature for 48 hours. Thereafter the material was crushed using pestle and mortar and finally sieved with a Retsch^{®} AS 200 Basic sieve shaker stack to obtain particles in the range of 50 - 500µm. The powder was tested using a spectrophotometer to confirm nicotine content.

300 mg of the resulting powder was mixed with 3.62 wt% of Ph. Eur grade anhydrous sodium carbonate and sealed in a pouch made of non-woven cellulose. The pH generated by a pouch in distilled water was then measured and compared to the pH obtained for seven different commercial products (Nils Cortado, ZYN^{®} Cool Mint Slim, VOLT^{®} Spearmint Breeze, ON! Berry, Nordic Spirit^{®} Elderflower, Lyft^{®} Ice Cool, and ZYN^{®} Cool Mint Mini Dry).

### Analysis

### Nicotine analysis:

300 mg of the powder was diluted in 100 mL of distilled water and shaken for about 1 min. The dilute was then analysed in a UV-spectrophotometer system (VWR UV-3100PC) at 260 nm wavelength.

### pH analysis:

The pH measurements were performed using a calibrated Mettler Toledo^{®} Seven compact pH/Ion S220 system with a Mettler Toledo^{®} InLab Expert Pro electrode. Each pouch was placed in a beaker with 25mL of distilled water and stirred for 30 min using a magnet and magnetic stirrer. After 30 min the magnet and pouch were removed and the pH measured.

### Results

### Nicotine analysis:

100% of the added nicotine was recovered in the sample

### pH analysis:

The pH results are summarised in Table 1 below.

**Table 1 - pH results**

| **Product** | **pH** |
|---|---|
| Chemically bonded ceramic | 8.418 |
| Nils Cortado | 8.268 |
| ZYN^{®} Cool Mint Slim | 7.425 |
| VOLT^{®} Spearmint Breeze | 8.687 |
| ON! Berry | 8.442 |
| Nordic Spirit^{®} Elderflower | 8.930 |
| Lyft^{®} Ice Cool | 8.528 |
| ZYN^{®} Cool Mint Mini Dry | 8.726 |

| | |
|---|---|
| Nils Cortado contains bamboo fiber and microcrystalline cellulose (MCC) as fillers. Zyn^{®} cool mint Slim uses MCC and plantfibre as fillers. Volt^{®} uses MCC as filler. On!^{®} Uses MCC and Maltitol as fillers. Nordic Spirit^{®} uses Poliacrilex, which is a gum base with nicotine. Lyft^{®} uses MCC as filler. | |

It can be concluded that the pH of the chemically bonded ceramic system is in line with the commercial products.

### Example 5 - Loaded powder for nicotine pouches

The following illustrates a representative intraoral formulation containing a nicotine-loaded powder made according to the method of the invention.

**Table 2 - Intraoral formulation**

| **Ingredient** | **Quantity (%w/w)** |
|---|---|
| Nicotine-loaded base powder | 91.9 |
| Sucralose | 0.1 |
| Sodium carbonate | 3.2 |
| Sodium chloride | 1.8 |
| NP Ice Cool (Flavour) | 3.0 |

### Method

The nicotine-loaded base powder was made using BP and DAB grade calcium sulphate hemihydrate (CaSH), and nicotine in the form of USP grade nicotine bitartrate dihydrate in according to the following method:
170.4 g of Nicotine Bitartrate salt was dissolved in 1215.7 g of de-ionized water in a household blender. 1519.6 g of CaSH was added in portions. The mix was blended for about 3 min using medium speed on the blender. Thereafter the blend was poured onto a silicon canvas with metal walls and left to set, creating a roughly 10mm thick plate. The plate was then put onto a metal plate with holes and left to dry at room temperature for roughly 24h. The casted plate was then broken up into smaller pieces using pestle and mortar and finally ground in a household flour mill. The resulting powder was then sieved using a Retsch^{®} AS 200 Basic sieve shaker stack to obtain particles in the range of 50 - 500µm. The resulting powder ("Nicotine-loaded base powder") was then mixed with the rest of the components according to Table 2 above. The mixing was performed by weighing the components into a glass vessel with lid and mixing for 1 hour in a WAB Turbula^{®} mixer at 60 rpm. After mixing, a portion of the powder mixture was filled and sealed in three separate non-woven fabric pouches using a pouching machine. Roughly 0.3g of powder was filled in each pouch.

### Analysis

The pouches was suspended in a steelwire "cage" in a beaker with 50mL of deionised water. A magnet was added to the beaker that was immediately placed on a magnetic stirrer.

### pH

The pH measurements were performed using a calibrated Mettler Toledo^{®} Seven compact pH/Ion S220 system with a Mettler Toledo^{®} InLab Expert Pro electrode. The pH-meter was set to automated measurements and the pH was measured once every minute.

### Nicotine release

Samples was withdrawn manually using a pipette and the samples were then analysed with a UV-spectrophotometer (VWR UV-3100PC) at a wavelength of 260 nm. Standard curves were obtained using defined solutions with nicotine base.

### Results

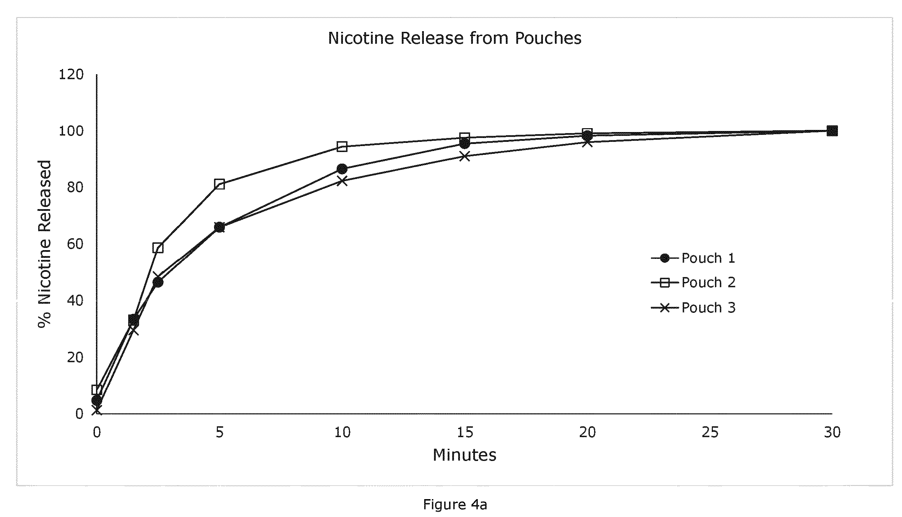

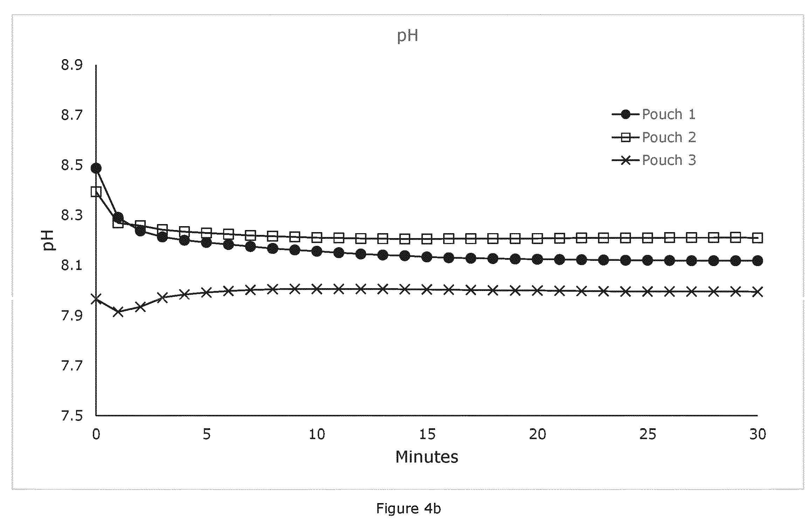

The resulting curves from Nicotine release and pH measurements can be seen for each of the three pouches in Figs 4a and 4b.

### Example 6 - Moist nicotine pouches

A moist formulation was created by mixing the powder formulation from Example 5 with 17wt% Propylene Glycol (PG). 0.6g of the powder from Example 5 was mixed with 0.1 g of PG in a beaker using a spatula. The resulting mixture had a texture and consistency similar to that of a commercial moist product.

### Analysis

Nicotine release and pH was tested according to the methods of Example 5.

### Results

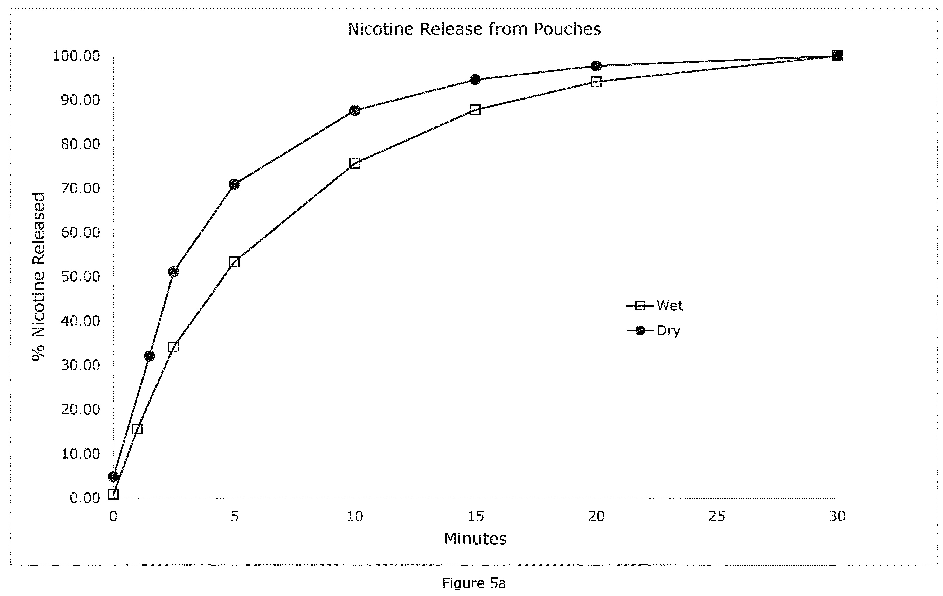

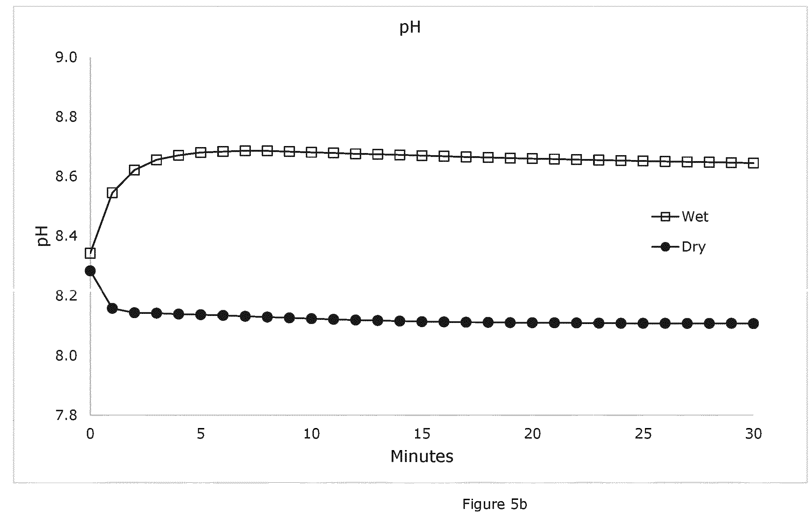

The results for nicotine release and pH are shown in Figs 5a and 5b where this "moist" formulation is compared to the dry formulation of Example 5. The data show that the nicotine release rates are comparable for both dry and moist formulations, confirming that both dry and moist formulations are capable of providing sustained release of nicotine over a meaningful timeframe.

### Example 7 - Taste evaluation

A small scale subjective evaluation with Nicotine pouch users (n=4) were performed. Each user was given a can with pouches produced according to Example 5 with two different flavours, one Citrus and one Mint (Powder 1 and Powder 2), as shown in Table 3:

**Table 3**

| **Ingredient** | **Quantity (%w/w)** | |
|---|---|---|
| | **Powder 1 (Citrus)** | **Powder 2 (Mint)** |
| Nicotine-loaded base powder | 91.9 | 91.9 |
| Sucralose | 0.1 | 0.1 |
| Sodium carbonate | 3.2 | 3.2 |
| Sodium chloride | 1.8 | 1.8 |
| NP Ice Cool (Flavour) | - | 3.0 |
| Citrus (Flavour) | 3.0 | - |

Each subject was also given one can of Dry and one can of Moist commercial competitive product (Zyn^{®} Mini dry and Zyn^{®} Slim, respectively) with Mint flavour for comparison. The subjects were asked to rate the smell in the can, the speed of nicotine onset, the duration of nicotine release, the speed of flavour onset and the duration of flavour release.

The results were given in terms of comparison to the dry and moist commercial products

### 1. Smell when opening the can:

Powders 1 and 2 had a better and more rich smell compared to the commercial dry product and similar to the commercial moist product.

### 2. Nicotine onset:

The commercial moist product had the fastest onset of nicotine. Powders 1 and 2 were equal to or faster than the commercial dry product.

### 3. Duration of nicotine release:

Powders 1 and 2 had a much longer nicotine release than the commercial dry product and a longer nicotine release than the commercial moist product.

### 4. Flavour onset:

The flavour for powders 1 and 2 could be felt and was fully developed much faster than for the commercial dry product. The performance of the commercial moist product was somewhere in between the dry commercial product and Powders 1 and 2.

### 5. Flavour duration

The duration of flavour release was much longer for Powders 1 and 2 than for both the dry and moist commercial products. The moist commercial product had a longer flavour duration than the dry product. Powders 1 and 2 released flavour for at least 45 - 60 minutes, long after the nicotine had been released. One subject even kept the pouch with Powder 1 in the mouth for as long as three hours and still had a good flavour from the pouch. The dry commercial product typically lost all flavour within 10 minutes and the moist commercial product within 15 - 20 minutes.

### Example 8 - Storage stability

The stability of a formulation of the invention under humid conditions was assayed using the following method.

### Test material

Test formulations of the invention (referred to as "Bioceramic powder") were made according to the method in Example 5. Commercial dry and moist formulations were used for comparison. The formulations were provided in the form of powders for storage which were then sealed in pouches made of non-woven cellulose prior to storage.

### Storage Conditions

The sealed pouches were stored in under the following conditions:
Test duration: 34 days.
Vessel/container: in open pouch cans without lid. Cans placed in larger sealed plastic containers with different humidity.
Temperature: room temperature. Typically varying between 20 °C and 23 °C.
Humidity: Two different levels of relative humidity ("RH") were used: 33% and 60% RH. The RH was controlled by having saturated salt solutions with different salts in the bottom of the plastic containers, as described in Greenspan L., Journal of Research of the National Bureau of Standards, vol. 81A, No. 1, 1977, 89-96.

### Analysis

Analysis of the nicotine content before and after storage was made using the UV-spectrophotometry method in Example 5. pH measurements were made using the method in Example 5.

### Results:

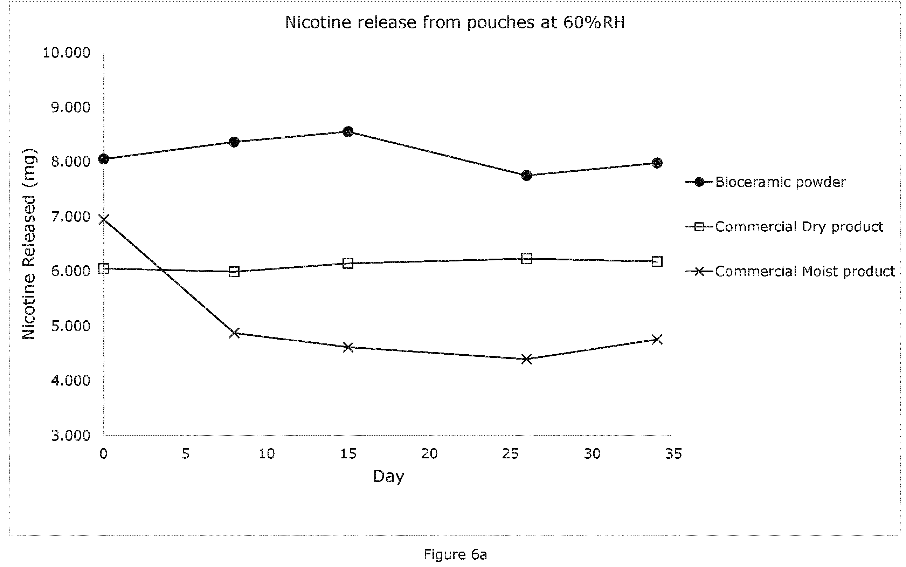

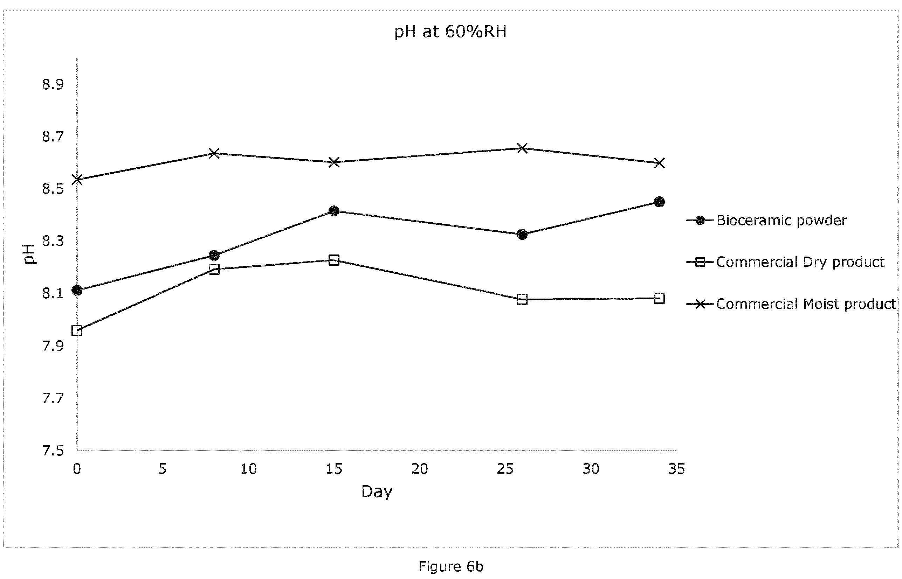

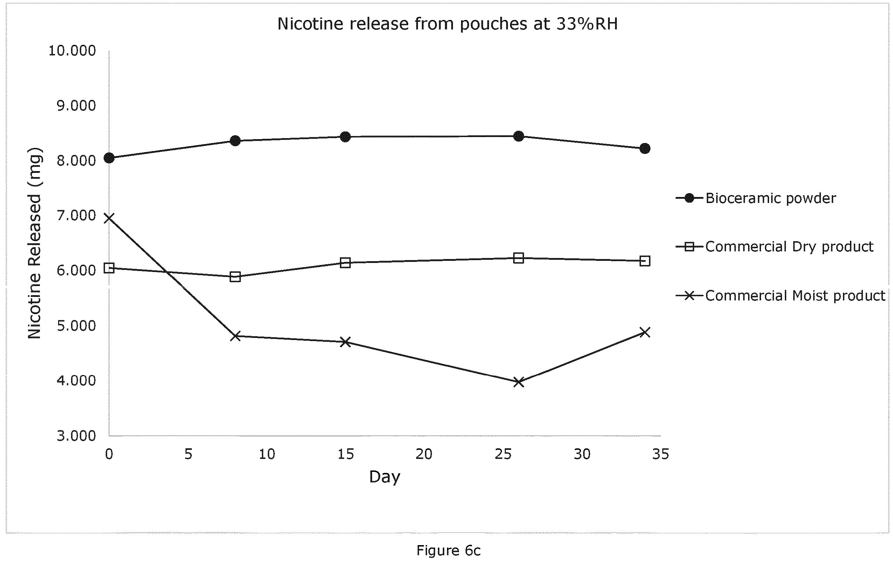

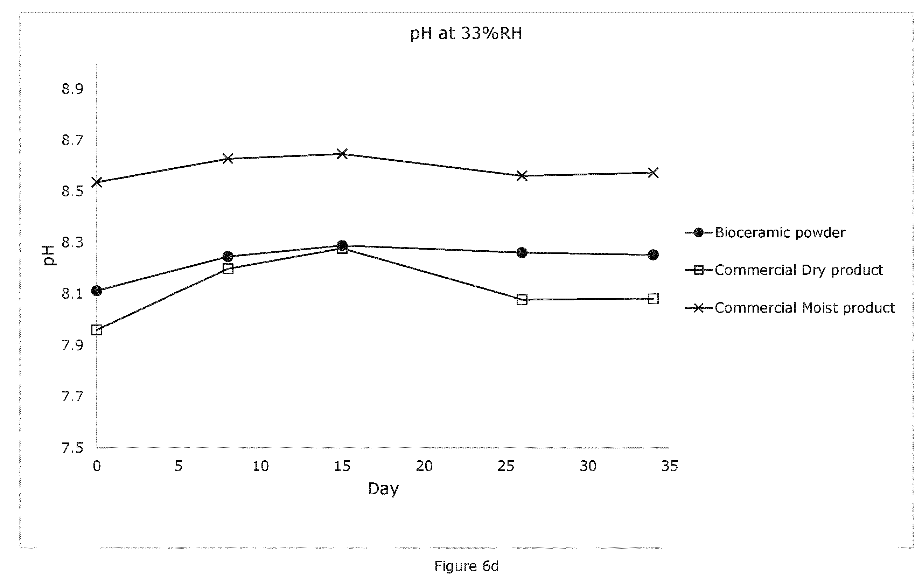

The nicotine release and pH results are shown in Fig 6a through 6d for 33% and 60% RH. In these figures, "Bioceramic powder" refers to test results for pouches containing the test formulation of the invention. The results show that, after 4 weeks of storage, the nicotine releasable from nicotine pouches containing the formulation of the invention was high. This indicates that formulations of the invention have storage stability properties (in terms of nicotine release under the tested conditions) that are as good as those of the dry commercial product, and are better than those for the moist commercial product.

### Example 9 - different carrier and pH regulating agent

This example illustrates using Calcium Silicate, Portland cement and sodium carbonate as pH regulating agent added to a Calcium Sulphate based nicotine powder.

### Materials

USP grade nicotine bitartrate dihydrate
De-ionised water
(CSH) BP, DAB grade Calcium sulphate Hemihydrate
(CS) Calcium Silicate powder (CaO / SiO2 = 1/1)
(PC) Portland cement
(SC) Sodium Carbonate

### Method

A nicotine-loaded base powder was made using BP and DAB grade calcium sulphate hemihydrate (CaSH), and nicotine in the form of USP grade nicotine bitartrate dihydrate in according to the following method:
170.4 g of Nicotine Bitartrate salt was dissolved in 1215.7 g of de-ionized water in a household blender. 1519.6 g of CaSH was added in portions. The mix was blended for about 3 min using medium speed on the blender. Thereafter the blend was poured onto a silicon canvas with metal walls and left to set, creating a roughly 10mm thick plate. The plate was then put onto a metal plate with holes and left to dry at room temperature for roughly 24h. The casted plate was then broken up into smaller pieces using pestle and mortar and finally ground in a household flour mill. The resulting powder was then sieved using a Retsch^{®} AS 200 Basic sieve shaker stack to obtain particles in the range of 50 - 500µm. The resulting powder ("Nicotine-loaded base powder") was then mixed with the CS and PC, respectively, to generate two different powders. In addition one powder was made where the alkaline agent used was the standard agent Sodium Carbonate, powder 3.
Powder 1: 1 g of Nicotine-loaded base powder was mixed with 5 wt% of CS
Powder 2: 1 g of Nicotine-loaded base powder was mixed with 3.5 wt% of PC
Powder 3: 1 g of Nicotine-loaded base powder was mixed with 3.5 wt% of SC

The powders were mixed by hand using a spatula in a beaker to create a homogenous blend. 0.3g of the resulting powders was respectively put into a standard size pouch made of non-woven material for analysis.

### Analysis

pH and nicotine release was performed according to methods described in Example 9.

### Results

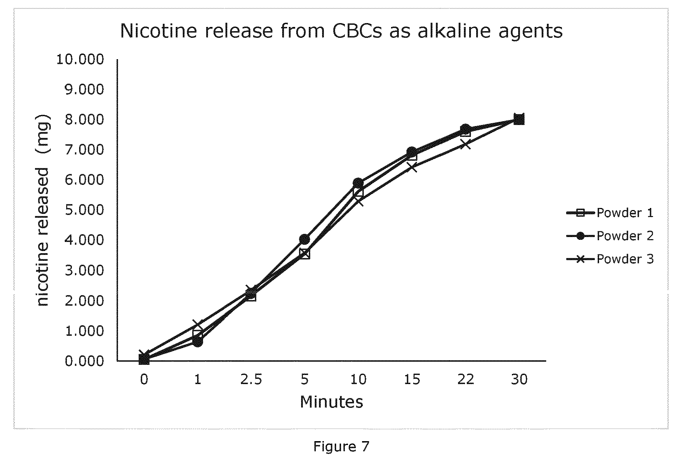

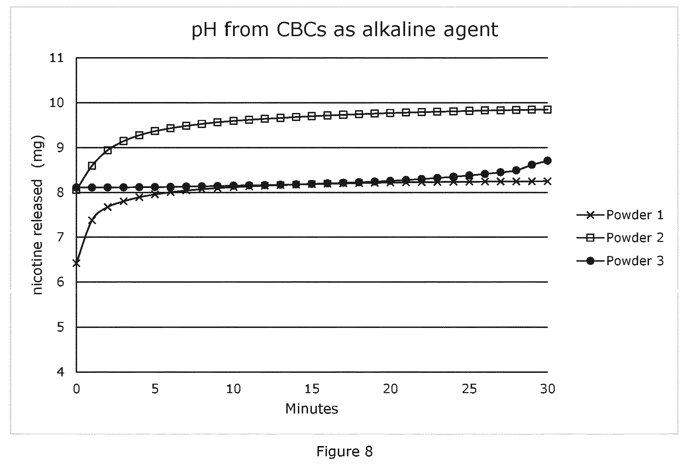

The resulting nicotine release and pH profiles can be seen in Figs 7 and 8, respectively.

### Example 10 - Real time stability of CBC based nicotine pouches

The nicotine content of nicotine pouches containing powders of the invention has been measured following storage under different conditions.

### Sample preparation

Three different types of samples were tested according to Table 4. They vary in manufacturing method, storage and analysis method.

**Table 4: The different samples tested**

| **Sample No** | **Powder Manufacturing / Flavour** | **Pouching** | **Storage condition** | **Analysis** | **Measured Time points** |
|---|---|---|---|---|---|
| 1 | Lab / Mint | Lab | In can at ambient room temp | UV-Spectrophotometer | 0 and 12 months |
| 2 | Lab / Citrus | CMO | Plastic box at ambient room temp | UV-Spectrophotometer and HPLC | 0 and 9 months |
| 3 | Lab / Mint | CMO | Plastic box at ambient room temp | UV-Spectrophotometer and HPLC | 0 and 9 months |
| 4 | Lab / Citrus | CMO | Can following user* | UV-Spectrophotometer and HPLC | 0 and 10 months |

| | | | | | |
|---|---|---|---|---|---|
| *The can with pouches was kept in handbag that followed the user around and was also opened from time to time | | | | | |

The manufacturing was made in steps first producing a nicotine-containing chemically bonded ceramic (CBC) powder, mixing the resulting powder with further ingredients and filling into pouches.

### Step 1

42.6 g USP grade Nicotine bitartrate dihydrate was dissolved in 312 g de-ionised water. 380.2 g BP, Dab grade Calcium sulphate hemihydrate was weighed into a bowl. The water - nicotine salt solution was added to the calcium sulphate hemihydrate and manually stirred to a homogeneous paste. The proportions of water - nicotine salt solution and calcium sulphate hemihydrate were selected to product a dry powder with a nicotine (free base) content of approximately 25-30 mg per gram of powder. The paste was poured onto an Al-foil sheet and left to set for about 10 minutes. Thereafter the set material was dried in a convection oven at 24 °C for approximately 24 hours. Subsequently the hardened material was crushed manually, using a pestle and mortar whereafter the crushed material was sieved to get a powder in the particle size range of 50 - 500 µm.

### Step 2

The nicotine-containing CBC powder from step 1 was mixed with other ingredients to produce a finished formulation according to Table 5. Mixing of the components were achieved by dry mixing in a Turbula^{®} dry mixer.

**Table 5: Components mixed to obtain final nicotine formulation.**

| **Ingredient** | **Quantity (%w/w)** |
|---|---|
| Nicotine-loaded base powder | 91.7 |
| Sucralose | 0.1 |
| Sodium carbonate | 3.3 |
| Sodium chloride | 1.9 |
| Flavour | 3 |

### Step 3

### Pouching in lab:

Pouches of non-woven heat sealable material designed for use as pouches was filled with 0.3g of the powder. The pouches were sealed using a standard heat sealer.

### Pouching at CMO:

The finished powders were sent to a contract manufacturing organisation (CMO) to be filled into pouches using a pouching machine designed for nicotine pouch filling. The pouch material used was a non-woven heat sealable material designed for use as pouches. Each pouch was filled with approximately 0.3 g of the final nicotine formulation.

### Analysis

To measure nicotine release, each pouch was suspended in a metal net cage in 100ml of distilled water in a glass beaker containing a stirrer magnet. The beaker was placed on a magnetic stirrer at a stirrer speed of 360rpm. Samples were taken out after 30 min using a pipette and put into sealable vials for analysis either in a UV-spectrophotometer or HPLC system.

### UV-spectrophotometer

The absorbance of the analytes was measured using a UV-spectrophotometer system (VWR UV-3100PC) at 260 nm wavelength. The absorbance of each analyte was calculated as nicotine concentration using a calibration curve and the known powder weight in each pouch.

### HPLC

The samples were analysed in a Shimadzu Prominence-I LC-2030 HPLC system with a UV detector.

### Results

The results are summarised in Table 6. The nicotine pouches had a good stability for the time periods and conditions measured.

**Table 6 - stability results**

| Sample | Nicotine content (~0.3g powder) at Time 0 | Nicotine content (~0.3g powder) / Time (months) | Analysis method |
|---|---|---|---|
| 1 | 7.6 mg | 7.7mg / 12 months | UV-Spectrophotometer |
| 2 | 7.3 mg | 6.8 mg / 9 months | UV-Spectrophotometer |
| 2 | 7.3 mg | 6.8 mg / 9 months | HPLC |
| 3 | 6.9 mg | 7.5 mg / 9 months | UV-Spectrophotometer |
| 3 | 6.9 mg | 7.6 mg / 9 months | HPLC |
| 4 | 7.3 mg | 6.2 mg / 10 months | UV-Spectrophotometer |
| 4 | 7.3 mg | 6.6 mg / 10 months | HPLC |

The analyses have some obvious sources of variation.
- The analytical methods themselves have inherent variation.
- The time period between measuring calibration curves and sample measurement may vary.
- The weight of the CMO filled pouches is not exact. The weight is taken as a mean of several pouches that was emptied from the same batch but not for the exact pouches that was measured, since an exact measurement requires the pouches to be emptied.

Nevertheless, the results are indicative of minimal loss over the time period and conditions studied.

### Example 11 - Portland Cement product

Hardened Portland cement was used as pH regulator with a calcium phosphate nicotine granulate.

### Method

### Sample preparation

### Step1:

### Manufacturing of Calcium phosphate nicotine granules

153 g USP grade Nicotine bitartrate dihydrate was dissolved in 430 ml of distilled water. 1973 g of DAB /BP grade Calcium sulphate hemihydrate was weighed into the steel drum of an intensive mixer type granulator. The granulator was started at a speed of 20 m/s and the nicotine / water solution was poured in during 15 s. The granulator was on for another 15 s whereafter the speed was lowered to half and run for another 120 s before turned off and the material was checked. The granulator was turned on again at 20 m/s speed, another 100 ml of water was added, and the machine run for 15 s and turned off. The granulator was started again, another 150 ml of water added, and the granulator was on for 15 s. The resulting granulate was poured onto an Al-tray and dried at room temperature for 24 hours. The dried granulate was sieved using a Retsch^{®} AS 200 Basic sieve shaker stack to obtain particles in the range of 50 - 500µm. The resulting nicotine content in the powder was approximately 6 mg of nicotine / 0.3 g of powder.

Step 2:

### Manufacturing of the hardened Portland cement powder

10 Portland cement and 4g water was mixed manually in a glass beaker into a homogeneous paste. The paste was then left to set and harden on an Al-foil at ambient room conditions for at least 1 hour. Thereafter the material was crushed manually using a pestle and mortar. The crushed powder was sieved using a Retsch^{®} AS 200 Basic sieve shaker stack to obtain particles in the range of 50 - 500µm.

### Step 3:

Mixing of Hardened Portland cement with Nicotine containing Calcium Phosphate granules.

20 g of the granules from step 1 was mixed with four different amounts of hardened Portland cement powder according to Table 7. Mixing was done in a Turbula^{®} dry mixer for 30 min at 50 rpm.

**Table 7: Four powders was prepared with varying wt% of hardened PC**

| Sample | Wt % hardened PC |
|---|---|
| Powder 1 | 1 |
| Powder 2 | 2 |
| Powder 3 | 3 |
| Powder 4 | 5 |

### Step 4

### Filling powder into pouches

0.3 g of powder was filled into pouches of a non-woven heat sealable material designed for use as nicotine pouches. Three pouches for powders 2, 3 and 4 in Table 7 were made.

### Analysis

Three different sets of measurements were performed with the different samples, two different pH measurements and one dissolution experiment.

### pH method 1:

0.3g of powders 1, 2 and 4, according to Table 7 was added to 100 ml of de-ionised water in a glass beaker with a stirring magnet. The beaker was placed on a magnetic stirrer at 360 rpm. After 30 minutes pH was measured using a calibrated Mettler Toledo Seven compact pH/Ion S220 system with a Mettler Toledo InLab Expert Pro electrode.

Three samples of each powder were analysed.

### pH method 2:

The filled pouches, with powders 2, 3 and 4 was suspended in a metal net cage in 100ml of de-ionised water in a glass beaker containing a stirrer magnet. The beaker was placed on a magnetic stirrer at a stirrer speed of 360 rpm. After 30 minutes pH was measured using a calibrated Mettler Toledo Seven compact pH/Ion S220 system with a Mettler Toledo InLab Expert Pro electrode. Three samples of each powder were analysed.

### Dissolution:

Pouches filled with 0.3 g of powder 3 according to Table 7, were suspended in a metal net cage in 100ml of de-ionised water in a glass beaker containing a stirrer magnet. The beaker was placed on a magnetic stirrer at a stirrer speed of 360 rpm. For dissolution analysis samples were taken after 30 minutes using a pipette and put into sealable vials for analysis. The samples were then analyzed by measuring absorbance using a UV-spectrophotometer system (VWR UV-3100PC) at 260 nm wavelength. The absorbance of each analyte was calculated as nicotine concentration using a calibration curve and the known powder weight. Three pouches were analyzed.

### Results

The results of the pH measurements are presented in Table 8 as a mean of three samples. A desirable pH of 8 or above could be achieved with powders 3 and 4.

**Table 8: Results from the two different pH methods**

| Material | pH (pH method 1) | pH (pH method 2) |
|---|---|---|
| Powder 1 | 5.4 | |
| Powder 2 | 7.6 | 7.6 |
| Powder 3 | | 8.1 |
| Powder 4 | 10.1 | 8.6 |

The results from the dissolution experiment can be seen in Table 9. It is confirmed that at least 6 mg of nicotine was dissolved from each sample confirming that the addition of hardened PC powder does not disturb the nicotine release.

**Table 9: Amount of dissolved nicotine in the dissolution experiment.**

| Pouch | Dissolved nicotine (mg) |
|---|---|
| 1 | 6.3 |
| 2 | 6.1 |
| 3 | 6.1 |

### Example 12 - Nicotine concentration in nicotine pouches after human use

Pouches containing bioceramic (chemically bonded ceramic) powders loaded with nicotine were produced and compared to commercially available nicotine pouches to evaluate the amount of nicotine remaining after 20 min of human use.

### Materials

USP grade nicotine bitartrate dihydrate
Distilled water
(CSH) BP, DAB grade Calcium sulphate Hemihydrate

### Manufacturing of CBC-based nicotine pouches

The manufacturing was made in steps first producing a nicotine-containing chemically bonded ceramic (CBC) granulate, mixing the resulting granulate with further ingredients, and then putting the mixture into pouches.

### Step 1:

153 g of nicotine bitartrate dihydrate was dissolved in 430 ml of distilled water. 1973 g of calcium sulphate hemihydrate was weighed into the steel drum of an intensive mixer type granulator. The granulator was started at a speed of 20 m/s and the nicotine / water solution was poured in during 15 s. The granulator was on for another 15 s whereafter the speed was lowered to half and run for another 120 s before turned off and the material was checked. The granulator was turned on again at 20 m/s speed, another 100 ml of water was added and the machine run for 15 s and turned off. The granulator was started again, another 150 ml of water added and the granulator was on for 15 s. The resulting granulate was poured onto an Al-tray and dried at room temperature for 24 hours. The dried granulate was sieved using a Retsch^{®} AS 200 Basic sieve shaker stack to obtain particles in the range of 50 - 200 µm.

### Step 2:

The nicotine-containing CBC granulate from step 1 was mixed with other ingredients to get a finished formulation according to Table 10. Mixing of the components was achieved by dry mixing in a Turbula^{®} dry mixer.

**Table 10: Components mixed to obtain final nicotine formulation**

| **Ingredient** | **Quantity** (%w/w) |
|---|---|
| Nicotine-loaded base powder | 86.7 |
| Sucralose | 0.1 |
| Sodium carbonate | 6.5 |
| Sodium chloride | 1.9 |
| NP Ice Cool (Flavour) | 2.8 |
| NP Peppermint flavor | 1.0 |
| NP cooling | 1.0 |

After mixing the powder was left to sit in a closed jar for at least 24 to let the flavors even out.

### Step 3:

Pouches of non-woven heat sealable material designed for use as pouches were filled with either 0.33g or 0.15g of the finished formulation. The pouches were sealed using a standard heat sealer. The pouches containing 0.15g were half the length of the pouches containing 0.33g, which was the same as the commercial ZYN^{®} products in size.

The molecular structure of the chemically bonded ceramic system obtained from this method was subjected to X-ray powder diffraction analysis and found to be the same as that obtained in Example 5. The following X-ray diffraction peaks of high intensity were observed for both hardened ceramics obtained from hydration of calcium sulfate hemihydrate: 11.5, 20.7, and 29.2 °2θ (using X-rays with a wavelength of 1.5406 Å). These are also the strongest peaks observable for calcium sulphate dihydrate.

### Human use testing

Two users ("User 1" and "User 2"), who currently use different brands of nicotine pouches, were allowed to use the different nicotine pouches according to the method below. The pouches used are described in Tables 11A and 11B. Zonnic is an approved nicotine replacement therapy (NRT) product rather than a product for recreational use.

**Tables 11A**

| Nicotine pouch | Product No. | Type of product | Weight material per pouch | Nicotine strength (mg per pouch) |
|---|---|---|---|---|
| CBC | 1 | Dry | 0.33 | 6 |
| CBC | 2 | Dry | 0.15 | 3 |
| Zyn Mini Dry Cool Mint (Swedish Match) | 3 | Dry | 0.4 | 6 |
| Zyn Mini Dry Apple Mint (Swedish Match) | 4 | Dry | 0.4 | 3 |
| Zyn Mini Dry Black Cherry (Swedish Match) | 5 | Dry | 0.4 | 3 |
| VELO Easy Mint mini | 6 | Moist | 0.5 | 4 |
| Zonnic | 7 | Dry | 0.17 | 4 |

**Table 11B - commercial formulations**

| **Product** | **Ingredients** |
|---|---|
| Zyn Mini Dry Cool mint | Filler (E 965, E460, E414), Acidity regulator (E500), Stabilizing agent (E463), Aromas, Nicotine, Sweetener (E950) |
| Zyn Mini Dry Apple mint | Filler (E 965, E460, E414), Acidity regulator (E500), Stabilizing agent (E463), Aromas, Nicotine, Sweetener (E950) |
| Zyn Mini Dry Black Cherry | Filler (E 965, E460, E414), Acidity regulator (E500), Stabilizing agent (E463), Aromas, Nicotine, Sweetener (E950) |
| VELO Easy Mint | Filler (460), Water, Flavour enhancer (Sodium Chloride), Xylitol, Aromas, Thickening agent (E401), Humectant (E 1520), Nicotine, Sweetener (E955), Acidity regulator (E 500), |
| Zonnic Mint 4mg | Filler (E460), Flavour, Antioxidation agent (E 304), Tri Sodium phosphate, Sweetener (E 950, E951)) |

To test a pouch the user placed the pouch between the upper lip and the gum and kept the pouch in place for twenty minutes without touching the pouch with the tongue. After twenty minutes the pouch was removed and immediately placed in a plastic vial and frozen to -20°C awaiting analysis. The users tested the pouches according to Table 12. A wash out period of at least 1 hour was mandatory between pouch uses.

**Table 12: Number of pouches tested per product and user**

| Product No. | 1 | 2 | 3 | 4 | 5 | 6 |
|---|---|---|---|---|---|---|
| User 1 | 6 | | 6 | | | |
| User 2 | | 6 | | 7 | 3 | 4 |

### Analysis

Before analysis, each used pouch was thawed at room temperature. The pouches were then suspended in a steel net cage in a beaker containing 100 ml of distilled water and a stirrer magnet. The assembly was immediately placed on a magnetic stirrer set to 360 rpm. After 60 min a sample was withdrawn and analysed using a UV-spectrophotometer system (VWR UV-3100PC) at 260 nm wavelength to give an absorbance value. Using calibration curves based on calibration solutions of Nicotine bi tartrate dissolved in distilled water the corresponding nicotine concentration could be calculated and subsequently the amount of nicotine remaining in the pouches after use could be calculated.

### Results

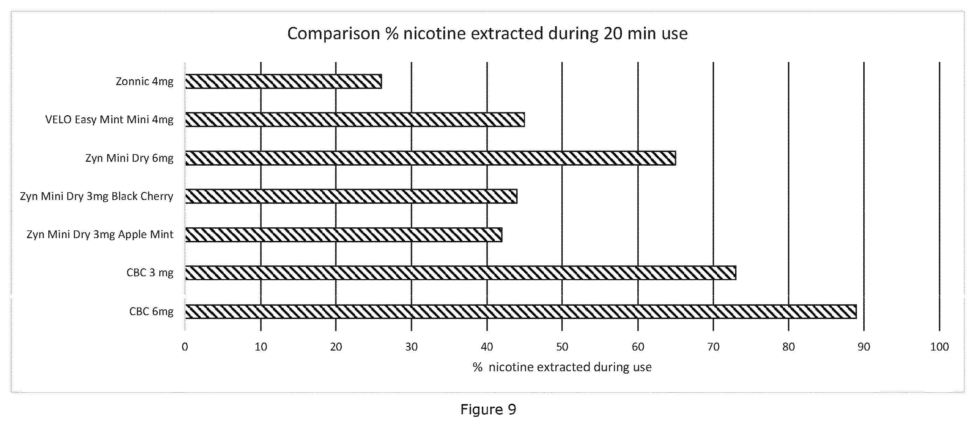

The results from the analysis are shown in Fig 9. The results clearly show that the CBC pouches have significantly less nicotine remaining in the pouches after use compared to the other products.

## Claims

1. An intraoral formulation comprising a solid, porous chemically bonded ceramic system, nicotine or a salt thereof, and a pH regulating agent, wherein the chemically bonded ceramic system contains calcium and is formed in the absence of said pH regulating agent, and wherein the chemically bonded ceramic system is formed in the presence of nicotine or a salt thereof such that said nicotine or salt thereof is located in pores in the chemically bonded ceramic system.

2. The formulation according to Claim 1, wherein the chemically bonded ceramic system is based on a calcium phosphate, a calcium sulphate, a calcium silicate, a calcium aluminate, or a mixture thereof.

3. The formulation according to Claim 2, wherein the chemically bonded ceramic system is formed from alpha-tricalcium phosphate, tetracalcium phosphate or calcium sulphate.

4. The formulation according to any one of the preceding claims, wherein the formulation is prepared using a salt of nicotine, such as nicotine bitartrate dihydrate.

5. The formulation according to any one of the preceding claims, wherein the pH regulating agent is a carbonate or a hydrogen carbonate or a phosphate compound.

6. The formulation according to any one of the preceding claims, wherein the formulation produces a pH of at least 8 when it is brought into contact with saliva.

7. The formulation according to any one of the preceding claims, wherein the pH regulating agent is predominantly located outside of the pores of the chemically bonded ceramic system.

8. The formulation according to any one of the preceding claims, wherein the chemically bonded ceramic system is present at from 40% to 98% by weight of the intraoral formulation.

9. A permeable, sealed bag containing a solid, porous chemically bonded ceramic system, nicotine or a salt thereof, and a pH regulating agent, wherein the chemically bonded ceramic system contains calcium and is formed in the absence of said pH regulating agent, and wherein the chemically bonded ceramic system is formed in the presence of nicotine or a salt thereof such that said nicotine or salt thereof is located in pores in the chemically bonded ceramic system.

10. The bag according to Claim 9, wherein the bag comprises a cavity surrounded by a permeable material, and the pH regulating agent is associated with the permeable material.

11. A sublingual tablet, buccal tablet, wafer or lozenge containing an intraoral formulation according to any one of Claims 1 to 8 and a bioadhesion and/or mucoadhesion promoting agent.

12. A method of forming an intraoral formulation comprising a solid, porous chemically bonded ceramic system, nicotine or a salt thereof, and a pH regulating agent, wherein the method comprises forming the chemically bonded ceramic system in the absence of said pH regulating agent and in the presence of nicotine or a salt thereof, and wherein the chemically bonded ceramic system contains calcium.

13. An intraoral formulation as defined in any one of Claims 1 to 8, a bag as defined in Claim 9 or 10, or a tablet as defined in Claim 11, for use in a method of treating nicotine dependence, treating one or more symptoms of nicotine dependence, aiding smoking cessation or ameliorating symptoms associated with a disease or conditions selected from the group consisting of dementia, Alzheimer's disease, Parkinson's disease, Huntington's disease and depression.

14. Use of an intraoral formulation as defined in any one of Claims 1 to 8, a bag as defined in Claim 9 or 10, or a tablet as defined in Claim 11, in the non-therapeutic intraoral administration of nicotine to a person.

## Patentansprüche

1. Intraorale Formulierung, umfassend ein festes, poröses, chemisch gebundenes keramisches System, Nikotin oder ein Salz davon und ein Mittel zur Regulierung des pH-Werts, wobei das chemisch gebundene keramische System Kalzium enthält und in Abwesenheit des Mittels zur Regulierung des pH-Werts gebildet wird und wobei das chemisch gebundene keramische System in Gegenwart von Nikotin oder einem Salz davon gebildet wird, sodass sich das Nikotin oder das Salz davon in Poren in dem chemisch gebundenen keramischen System befindet.

2. Formulierung nach Anspruch 1, wobei das chemisch gebundene keramische System auf einem Calciumphosphat, einem Calciumsulfat, einem Calciumsilikat, einem Calciumaluminat oder einer Mischung davon basiert.

3. Formulierung nach Anspruch 2, wobei das chemisch gebundene keramische System aus Alpha-Tricalciumphosphat, Tetracalciumphosphat oder Calciumsulfat gebildet ist.

4. Formulierung nach einem der vorstehenden Ansprüche, wobei die Formulierung unter Verwendung eines Nikotinsalzes, wie Nikotinbitartrat-Dihydrat, hergestellt wird.

5. Formulierung nach einem der vorstehenden Ansprüche, wobei das pH-regulierende Mittel ein Carbonat oder ein Hydrogencarbonat oder eine Phosphatverbindung ist.

6. Formulierung nach einem der vorstehenden Ansprüche, wobei die Formulierung einen pH-Wert von mindestens 8 ergibt, wenn sie mit Speichel in Kontakt gebracht wird.

7. Formulierung nach einem der vorstehenden Ansprüche, wobei sich das pH-regulierende Mittel überwiegend außerhalb der Poren des chemisch gebundenen keramischen Systems befindet.

8. Formulierung nach einem der vorstehenden Ansprüche, wobei das chemisch gebundene keramische System zu 40 bis 98 Gew.-% in der intraoralen Formulierung enthalten ist.

9. Durchlässiger, versiegelter Beutel, enthaltend ein festes, poröses, chemisch gebundenes keramisches System, Nikotin oder ein Salz davon und ein Mittel zur Regulierung des pH-Wertes, wobei das chemisch gebundene keramische System Kalzium enthält und in Abwesenheit des Mittels zur Regulierung des pH-Wertes gebildet wird und wobei das chemisch gebundene keramische System in Gegenwart von Nikotin oder einem Salz davon gebildet wird, sodass sich das Nikotin oder das Salz davon in Poren in dem chemisch gebundenen keramischen System befindet.

10. Beutel nach Anspruch 9, wobei der Beutel einen Hohlraum umfasst, der von einem durchlässigen Material umgeben ist, und das pH-regulierende Mittel mit dem durchlässigen Material verbunden ist.

11. Sublinguale Tablette, bukkale Tablette, Oblate oder Lutschtablette, enthaltend eine intraorale Formulierung nach einem der Ansprüche 1 bis 8 und ein die Bioadhäsion und/oder Mukoadhäsion förderndes Mittel.

12. Verfahren zur Bildung einer intraoralen Formulierung, die ein festes, poröses, chemisch gebundenes keramisches System, Nikotin oder ein Salz davon und ein Mittel zur Regulierung des pH-Wertes umfasst, wobei das Verfahren die Bildung des chemisch gebundenen keramischen Systems in Abwesenheit des Mittels zur Regulierung des pH-Wertes und in Gegenwart von Nikotin oder eines Salzes davon umfasst und wobei das chemisch gebundene keramische System Calcium enthält.

13. Intraorale Formulierung nach einem der Ansprüche 1 bis 8, ein Beutel nach Anspruch 9 oder 10 oder eine Tablette nach Anspruch 11 zur Verwendung in einem Verfahren zur Behandlung der Nikotinabhängigkeit, zur Behandlung eines oder mehrerer Symptome der Nikotinabhängigkeit, zur Unterstützung der Raucherentwöhnung oder zur Linderung von Symptomen im Zusammenhang mit einer Krankheit oder Zuständen, ausgewählt aus der Gruppe, bestehend aus Demenz, Alzheimer-Krankheit, Parkinsonkrankheit, Huntington-Krankheit und Depression.

14. Verwendung einer intraoralen Formulierung nach einem der Ansprüche 1 bis 8, eines Beutels nach Anspruch 9 oder 10 oder einer Tablette nach Anspruch 11 bei der nicht-therapeutischen intraoralen Verabreichung von Nikotin an eine Person.

## Revendications

1. Formulation intra-orale comprenant un système céramique solide, poreux, chimiquement lié, de la nicotine ou un sel de celle-ci, et un agent régulateur de pH, dans laquelle le système céramique chimiquement lié contient du calcium et est formé en l'absence dudit agent régulateur de pH, et dans laquelle le système céramique chimiquement lié est formé en présence de nicotine ou d'un sel de celle-ci de telle sorte que ladite nicotine ou ledit sel de celle-ci est situé dans les pores du système céramique chimiquement lié.

2. Formulation selon la revendication 1, dans laquelle le système céramique chimiquement lié repose sur un phosphate de calcium, un sulfate de calcium, un silicate de calcium, un aluminate de calcium ou un mélange de ceux-ci.

3. Formulation selon la revendication 2, dans laquelle le système céramique chimiquement lié est formé à partir de phosphate tricalcique alpha, de phosphate tétracalcique ou de sulfate de calcium.

4. Formulation selon l'une quelconque des revendications précédentes, dans laquelle la formulation est préparée à l'aide d'un sel de nicotine, tel que le bitartrate de nicotine dihydraté.

5. Formulation selon l'une quelconque des revendications précédentes, dans laquelle l'agent régulateur de pH est un carbonate, un hydrogénocarbonate ou un composé phosphate.

6. Formulation selon l'une quelconque des revendications précédentes, dans laquelle la formulation produit un pH d'au moins 8 lorsqu'elle est mise en contact avec la salive.

7. Formulation selon l'une quelconque des revendications précédentes, dans laquelle l'agent régulateur de pH est principalement situé à l'extérieur des pores du système céramique chimiquement lié.

8. Formulation selon l'une quelconque des revendications précédentes, dans laquelle le système céramique chimiquement lié est présent à hauteur de 40 % à 98 % en poids de la formulation intra-orale.

9. Sac perméable, scellé, contenant un système céramique solide, poreux, chimiquement lié, de la nicotine ou un sel de celui-ci, et un agent régulateur de pH, dans lequel le système céramique chimiquement lié contient du calcium et est formé en l'absence dudit agent régulateur de pH, et dans lequel le système céramique chimiquement lié est formé en présence de nicotine ou d'un sel de celui-ci de telle sorte que ladite nicotine ou ledit sel de celle-ci est situé dans les pores du système céramique chimiquement lié.

10. Sac selon la revendication 9, dans lequel le sac comprend une cavité entourée d'un matériau perméable, et l'agent régulateur de pH est associé au matériau perméable.

11. Comprimé sublingual, comprimé buccal, cachet ou pastille contenant une formulation intra-orale selon l'une quelconque des revendications 1 à 8 et un agent favorisant la bioadhésion et/ou la mucoadhésion.

12. Procédé de formation d'une formulation intra-orale comprenant un système céramique solide, poreux, chimiquement lié, de la nicotine ou un sel de celui-ci, et un agent régulateur de pH, dans lequel le procédé comprend la formation du système céramique chimiquement lié en l'absence dudit agent régulateur de pH et en présence de nicotine ou d'un sel de celui-ci, et dans lequel le système céramique chimiquement lié contient du calcium.

13. Formulation intra-orale telle que définie dans l'une quelconque des revendications 1 à 8, sac tel que défini dans la revendication 9 ou 10, ou comprimé tel que défini dans la revendication 11, destiné à être utilisé dans un procédé de traitement de la dépendance à la nicotine, de traitement d'un ou plusieurs symptômes de la dépendance à la nicotine, d'aide au sevrage tabagique ou d'atténuation des symptômes associés à une maladie ou à des affections sélectionnées dans le groupe constitué de démence, de la maladie d'Alzheimer, de la maladie de Parkinson, de la maladie de Huntington et de dépression.

14. Utilisation d'une formulation intra-orale telle que définie dans l'une quelconque des revendications 1 à 8, d'un sac tel que défini dans la revendication 9 ou 10, ou d'un comprimé tel que défini dans la revendication 11, dans l'administration intra-orale non thérapeutique de nicotine à une personne.
